# EUROPEAN PATENT SPECIFICATION

(11) **EP 1 824 536 B1**
(45) Date of publication and mention of the grant of the patent: **26.08.2009**
(21) Application number: 05819310.3
(22) Date of filing: 02.12.2005
(51) Int. Cl.: A61M 5/142

(54) **VENTILATED SKIN MOUNTABLE DEVICE**
BELÜFTETE AN DER HAUT BEFESTIGBARE VORRICHTUNG
SYSTEME VENTILE POUVANT ETRE MONTE SUR LA PEAU

(30) Priority: 06.12.2004 DK 200401893
(43) Date of publication of application: 29.08.2007
(73) Proprietor: NOVO NORDISK A/S, 2880 Bagsvaerd (DK)
(72) Inventor: ETHELFELD, Erik, Winkel, DK-1360 Copenhagen (DK); SCHMIDT, Nicolai, Michael, DK-2850 Nærum (DK); NIELSEN, John, Stern, DK-3450 Allerød (DK)
(86) International application number: PCT/EP2005/056430
(87) International publication number: WO 2006/061354

(56) References cited:
- WO-A-20/04087240
- US-A- 5 925 017
- US-A1- 2003 088 238
- US-A1- 2003 135 159
- US-A1- 2004 204 673

## Description

The present invention generally relates to a device which is adapted for application to a skin surface of a subject and comprises a transcutaneous device unit and an attachable process unit. In a specific aspect a transcutaneous drug delivery device is provided in combination with a drug delivery unit. In a further aspect a transcutaneous sensor device is provided in combination with a unit processing or transmitting data acquired from the sensor.

### BACKGROUND OF THE INVENTION

In the disclosure of the present invention reference is mostly made to the treatment of diabetes by injection or infusion of insulin, however, this is only an exemplary use of the present invention.

Portable drug delivery devices for delivering a drug to a patient are well known and generally comprise a reservoir adapted to contain a liquid drug and having an outlet in fluid communication with a hollow infusion needle, as well as expelling means for expelling a drug out of the reservoir and through the skin of the subject via the hollow needle. Such devices are often termed infusion pumps.

Basically, infusion pumps can be divided into two classes. The first class comprises infusion pumps which are relatively expensive pumps intended for 3-4 years use, for which reason the initial cost for such a pump often is a barrier to this type of therapy. Although more complex than traditional syringes and pens, the pump offer the advantages of continuous infusion of insulin, precision in dosing and optionally programmable delivery profiles and user actuated bolus infusions in connections with meals.

Addressing the above problem, several attempts have been made to provide a second class of drug infusion devices that are low in cost and convenient to use. Some of these devices are intended to be partially or entirely disposable and may provide many of the advantages associated with an infusion pump without the attendant cost and inconveniencies, e.g. the pump may be prefilled thus avoiding the need for filling or refilling a drug reservoir. Examples of this type of infusion devices are known from US patents 4,340,048 and 4,552,561 (based on osmotic pumps), US patent 5,858,001 (based on a piston pump), US patent 6,280,148 (based on a membrane pump), US patent 5,957,895 (based on a flow restrictor pump (also know as a bleeding hole pump)), US patent 5,527,288 (based on a gas generating pump), or US patent 5,814,020 (based on a swellable gel) which all in the last decades have been proposed for use in inexpensive, primarily disposable drug infusion devices. US patent 6,364,865 discloses a manually held infusion device allowing two vial-type containers to be connected and a pressure to be build up in one of the containers to thereby expel a drug contained in that container. US 2003/0088238 upon which the two-part form of claim 1 is based discloses a modular drug delivery system in which a control unit can be coupled to a skin-mountable reservoir unit.

The disposable pumps generally comprises a skin-contacting mounting surface adapted for application to the skin of a subject by adhesive means, and with the infusion needle arranged such that in a situation of use it projects from the mounting surface to thereby penetrate the skin of the subject, whereby the place where the needle penetrates the skin is covered while the appliance is in use. The infusion needle may be arranged to permanently project from the mounting surface such that the needle is inserted simultaneously with the application of the infusion pump, this as disclosed in US patents 2,605,765, 4,340,048 and in EP 1 177 802, or the needle may be supplied with the device in a retracted state, i.e. with the distal pointed end of the needle "hidden" inside the pump device, this allowing the user to place the pump device on the skin without the possibility of observing the needle, this as disclosed in US patents 5,858,001 and 5,814,020. In addition to pumps, alternative means for transporting a fluid drug may be used, e.g. iontophoresis as discussed below.

Although it can be expected that the above described second class of fully or partly disposable infusion devices can be manufactured considerably cheaper than the traditional durable infusion pump, they are still believed to be too expensive to be used as a real alternative to traditional infusion pumps for use on an every-day basis.

Before turning to the disclosure of the present invention, a different type of device relying on the insertion of a needle or needle-like structure will be described.

Although the above-described drug infusion pumps, either disposable or durable, may provide convenience of use and improved treatment control, it has long been an object to provide a drug infusion system for the treatment of e.g. diabetes which would rely on closed loop control, i.e. being more or less fully automatic, such a system being based on the measurement of a value indicative of the condition treated, e.g. the blood glucose level in case of insulin treatment of diabetes. In principle, such systems have been known for more than two decades, see for example US patent 4,245,634 which discloses an artificial beta cell for regulating blood glucose concentration in a subject by continuously analyzing blood from the patient and deriving a computer output signal to drive a pump which infuses insulin at a rate corresponding to the signal, however, mainly due to problems associated with the glucose sensors such systems have until today not been very successful. Although a closed loop system would be a desirable implementation of a given sensor system, such a sensor could also be utilized as a monitor system providing the patient with information for manually controlling treatment, e.g. insulin treatment by injections and/or infusion by pump.

A given monitor system for measuring the concentration of a given substance may be based on invasive or non-invasive measuring principles. An example of the latter would be a non-invasive glucose monitor arranged on the skin surface of a patient and using near-IR spectroscopy, however, the present invention is concerned with the introduction of a transcutaneous device such as a sensor element.

In recent years, a variety of electrochemical sensors have been developed for a range of applications, including medical applications for detecting and/or quantifying specific agents in a patient's blood. As one example, glucose sensors have been developed for use in obtaining an indication of blood glucose levels in a diabetic patient. As described above, such readings can be especially useful in monitoring and/or adjusting a treatment regimen which typically includes regular administration of insulin to the patient.

When a sensor element is introduced subcutaneously, the body responds to the element as an insult and produces a specialized biochemical and cellular response which may lead to the development of a foreign body capsule around the implant and consequently may reduce the flux of glucose to the sensor. Consequently, the percutaneous approach aims to acquire data during the first period of this tissue response.

The monitoring method can be of three types: non-reactive, reversibly reactive or irreversibly reactive. The type of sensor which, thus far, has been found to function most effectively in vivo is the amperometric sensor relying on irreversible, transport-dependent reactive glucose assays. For a detailed review of the different types of glucose sensors reference is made to Adam Heller, Implanted electrochemical glucose sensors for the management of diabetes, Annu. Rev. Biomed. Eng. 1999, 01:153-175.

The sensor may be placed subcutaneously being connected to external equipment by wiring or the substance (fluid) to be analysed may be transported to an external sensor element, both arrangements requiring the placement of a subcutaneous component, the present invention addressing both arrangements. However, for simplicity the term "sensor" is used in the following for both types of sensor elements.

Turning to the sensor elements *per se,* relatively small and flexible electrochemical sensors have been developed for subcutaneous placement of sensor electrodes in direct contact with patient blood or other extra-cellular fluid (see for example US patent 5,482,473, wherein such sensors can be used to obtain periodic or continuous readings over a period of time. In one form, flexible transcutaneous sensors are constructed in accordance with thin film mask techniques wherein an elongated sensor includes thin film conductive elements encased between flexible insulative layers of polyimide sheet or similar material. Such thin film sensors typically include exposed electrodes at a distal end for transcutaneous placement in direct contact with patient blood or other fluid, and exposed conductive contacts at an externally located proximal end for convenient electrical connection with a suitable monitor device.

Insertion devices for this type of sensors are described in, among others, US patents 5,390,671, 5,391,950, 5,568,806 and 5,954,643.

### DISCLOSURE OF THE INVENTION

Having regard to the above-identified problems, it is an object of the present invention to provide a skin mountable medical device or system and components therefore, which allow such a device or system to be used in a convenient and cost-effective manner. The configuration of the system and the components therefore should contribute in providing a medical drug delivery or sensor assembly which allow for easy and swift operation yet being reliable in use.

In the disclosure of the present invention, embodiments and aspects will be described which will address one or more of the above objects or which will address objects apparent from the below disclosure as well as from the description of exemplary embodiments.

Thus, corresponding to a first aspect, a medical device is provided comprising a transcutaneous device unit and a process unit. The transcutaneous device unit comprises a first housing, a lower surface adapted for application to a skin surface of a subject, and a transcutaneous device. The process unit comprises a second housing comprising a lower surface adapted to face towards the skin surface of the subject, and a process assembly. The first and second housings are adapted to be coupled to each other such that at least a portion of the lower surface of the second housing is free to move relative to the skin surface when the first unit is attached to the skin surface and the first and second housings are coupled to each other. The coupling may be substantially rigid. The transcutaneous device unit may comprise a flexible patch portion having an upper surface and a lower surface with an adhesive, wherein at least a portion of the patch faces the lower surface of the second housing and is free to move relative thereto when the units are coupled to each other.

In a second aspect, a medical device is provided comprising a transcutaneous device unit and a process unit. The transcutaneous device unit comprises a flexible patch portion comprising an upper surface and a lower surface, the lower surface being adapted for application to a skin surface of a subject, a first housing, and a transcutaneous device. The process unit comprises a second housing comprising a lower surface, and a process assembly. The first and second housings are adapted to be coupled to each other with the lower surface of the second housing facing towards at least a portion of the upper surface of the patch portion, such that at least a portion of the flexible patch portion facing towards the lower surface of the second housing is free to move relative thereto. The coupling may be substantially rigid.

In a further aspect, a medical device is provided comprising a transcutaneous device unit and a process unit. The transcutaneous device unit comprises a flexible patch portion with an upper surface and a lower mounting surface adapted for application to a skin surface of a subject, a first housing, and a transcutaneous device arranged in the housing. The process unit comprises a second housing with a lower surface and a process assembly. The first and second housings are adapted to be secured to each other in such a way that at least a portion of the flexible patch portion facing the lower surface of the second housing is allowed to bend freely relative to the housing. The coupling may be substantially rigid.

In this way the relatively flexible patch portion can adapt to the skin surface to which it is mounted both statically and dynamically without being restricted in its movements by the normally much stiffer process unit. For example, if the flexible patch is arranged on a curved body portion it will be able to conform to the curvature both initially and during movement. Further, as the process unit is moved relative to the flexible patch the space between the two units is vented, this preventing build-up of moisture or heat. For example, most materials used for forming skin attachable patches are permeable to moisture, however, when a process unit is arranged directly onto the patch this capability is restricted unless the space between the two units are properly ventilated. To reduce the risk that the process unit is "caught" and thus pulled off the skin as it is lifted away from the patch portion it may be desirable to provide the patch portion with a degree of rigidity, e.g. by incorporation of stiffening structures, allowing only the flexibility necessary to provide the desired level of comfort and functionality. Alternatively, the lower surface of the process unit may fully or partly face directly against the skin, i.e. with no patch portion interposed, this allowing the space between the process unit and the skin to be vented as the process unit during use will be allowed to move relative to the skin.

In an exemplary embodiment a medical device is provided comprising a transcutaneous device unit and a process unit. The transcutaneous device unit comprises a flexible patch portion with upper and lower surfaces the lower surface being adapted for application to a skin surface of a subject, a first housing comprising a first coupling, and a transcutaneous device arranged in the housing. The process unit comprises a second housing with a lower surface and a second coupling arranged at a peripheral portion of the second housing, and a process assembly. The first and second couplings are adapted to be connected to each other with the upper surface of the patch facing towards the lower surface of the second housing, such that the flexible patch portion facing towards the lower surface of the second housing is substantially free to move relative thereto. For most applications, the two couplings will provide a substantially rigid connection between the two housings.

To enhance venting between the process unit and the patch the lower surface of the second housing and the upper surface of the patch may be formed to prevent full engagement between the two surfaces when they are arranged against each other. For example, at least one of the two units may be provided with a raised portion providing a free space between the two units when they are arranged against each other. To further enhance venting the patch portion facing towards the second housing may comprise one or more cut-out portions whereby a portion of the lower surface of the second housing in a situation of use faces the skin surface of the subject to which the medical device is arranged.

In an exemplary embodiment the transcutaneous device unit comprises a flexible sheet member with an upper and a lower surface, the lower surface being provided with a medical grade adhesive allowing the transcutaneous device unit to be attached to a skin surface of a subject, wherein the first housing comprises a lower surface attached to the upper surface of the flexible sheet member, e.g. by adhesive or by welding. The sheet may be from a woven or non-woven material or from a laminate thereof and preferably possesses good breathability providing a high degree of wearer comfort. One or more layers, e.g. the layer providing the upper surface, may be weldable allowing for attachment of the first housing by welding.

To support the first housing and improve its attachment to the sheet material one or more support members may be provided extending from the first housing, the support member being attached to the upper surface of the sheet member. In this way such a support member may serve also as a raised element as disclosed above.

In an exemplary embodiment the transcutaneous device unit comprises a transcutaneous drug delivery device, and the process unit comprises a reservoir adapted to contain a fluid drug, and an expelling assembly adapted for cooperation with the reservoir to expel fluid drug out of the reservoir and through the skin of the subject via the transcutaneous drug delivery device when the two units are coupled to each other.

The transcutaneous drug delivery device (which term also covers the similar terms transcutaneous access device and transcutaneous access tool traditionally used in this technical field) may be in the form of e.g. a pointed hollow infusion needle, a micro needle array, or a combination of a relatively flexible per se blunt cannula with a pointed insertion needle may provide a pointed transcutaneous device, the insertion needle being retractable after insertion of the blunt portion of the transcutaneous device. In the latter case the portion of the transcutaneous device actually placed in the subject does not necessarily comprise a pointed end allowing the combined transcutaneous device to be inserted through the skin, such a pointed end being withdrawn during insertion of the transcutaneous device. The cannula is advantageously soft and flexible relative to the insertion needle which typically is a solid steel needle. In the disclosure of the present invention as well as in the description of the exemplary embodiments, reference will mostly be made to a transcutaneous device in the form of an infusion needle. The length of the transcutaneous device may be chosen in accordance with the actual application, e.g. a hollow steel needle which may be inserted at a substantially right angle relative to the skin surface may have an inserted length of 2-8 mm, preferably 3-5 mm, whereas a cannula which may also be inserted at an oblique angle relative to the skin surface may be somewhat longer, e.g. 4-20 mm. Indeed, the first housing may comprise more than one transcutaneous drug delivery device.

The transcutaneous device unit may be supplied with e.g. a needle, soft cannula or sensor projecting from the mounting surface, however, to limit the risk of accidental needle injuries, the pointed end of the transcutaneous device is advantageously moveable between an initial position in which the skin-penetrating end is retracted relative to the mounting surface, and an extended position in which the pointed end projects relative to the mounting surface. Depending on the intended method of mounting the device on the user, the transcutaneous device may be moved between the two positions as the two units are connected to each, as would be appropriate in case the transcutaneous device unit is mounted on the skin of the user before the reservoir unit is connected. However, in case the two units are intended to be connected to each other before assembled units are mounted on the skin of the user, the transcutaneous device unit advantageously comprises user-actuatable actuation means for moving the pointed end of the transcutaneous device between the initial and the extended position.

To further reduce the likelihood of transcutaneous device injuries, the skin-penetrating end of the transcutaneous device may be moveable between the extended position in which the skin-penetrating end projects relative to the mounting surface, and a retracted position in which the skin-penetrating end is retracted relative to the mounting surface. Correspondingly, the combined device may comprise user-actuatable retraction means for moving the skin-penetrating end of the transcutaneous device between the extended and the retracted position when the retraction means is actuated. To prevent re-use of the transcutaneous device, the transcutaneous device may be permanently locked in its retraced position.

The transcutaneous drug delivery device may also be in the form of a transcutaneous device comprising no skin penetrating elements, e.g. a jet injection device or electrodes allowing an ionic agent to permeate from a predetermined site on the surface of skin into the subcutaneous tissue of the subject by using the principle of iontophoresis. For a more thorough discussion of iontophoresis reference is made to US patent 6,622,037.

The term expelling assembly covers an aggregation of components or structures which in combination provides that a fluid can be expelled from the reservoir. The expelling assembly may e.g. be a mechanical pump (e.g. a membrane pump, a piston pump or a roller pump) in combination with electronically controlled actuation means, a mechanically driven pump (e.g. driven by a spring), a gas driven pump or a pump driven by an osmotic engine. The expelling assembly may also me in the form of an aggregation of components or structures which in combination provides that a fluid can be expelled from the reservoir when the expelling assembly is controlled or actuated by a controller external to the expelling assembly. Depending on the nature of the transcutaneous device the expelling assembly may be of different configuration and nature. For example, when one or more hollow infusion needles or cannulas are used, the expelling assembly may be arranged to force or suck the fluid drug from the reservoir, whereas in the case of iontophoresis the expelling means would be means for applying a current over a set of electrodes, i.e. "driving" means.

In a further exemplary embodiment the transcutaneous device unit comprises a transcutaneous sensor device and the process unit is adapted to transmit and/or process data acquired via the sensor. As with the transcutaneous drug delivery device, a sensor device may be non-penetrating or penetrating. A non-penetrating sensor may allow a body parameter to be sensed in the subcutaneous space, e.g. by using a light source and light detector, or by transporting fluid from the subcutaneous space to the skin surface, e.g. by applying a current across the skin surface. A penetrating sensor may allow a body parameter to be sensed in the subcutaneous space, e.g. by using a needle formed sensor as discussed in the introduction, or by transporting fluid from the subcutaneous space to detection assembly by means of a conduit, this principle being known as micro-dialysis. An example of a penetrating needle-sensor and a corresponding process unit is shown in US patent 6,809,653 which discloses a characteristic monitor system including a data receiving device, a transcutaneous needle sensor for producing signal indicative of a characteristic of a subject (e.g. a blood glucose value), and a processor device. The processor device includes a housing, a sensor connector, a processor, and in the shown embodiment a transmitter. In the shown embodiment the processor coupled to the sensor processes the signals from the sensor for transmission to the remotely located data receiving device, however, the processed data could also be shown directly on a display provided on the processor device. The data receiving device may be a characteristic monitor, a data receiver that provides data to another device, a wireless programmer for a medical device (e.g. a remote control), a medication delivery device (such as an infusion pump), or the like.

As used herein, the term "drug" is meant to encompass any drug-containing flowable medicine capable of being passed through a delivery means such as a hollow needle in a controlled manner, such as a liquid, solution, gel or fine suspension. Representative drugs include pharmaceuticals such as peptides, proteins, and hormones, biologically derived or active agents, hormonal and gene based agents, nutritional formulas and other substances in both solid (dispensed) or liquid form. In the description of the exemplary embodiments reference will be made to the use of insulin. Correspondingly, the term "subcutaneous" infusion is meant to encompass any method of transcutaneous delivery to a subject. Further, the term needle (when not otherwise specified) defines a piercing member adapted to penetrate the skin of a subject.

### BRIEF DESCRIPTION OF THE DRAWINGS

In the following the invention will be further described with references to the drawings, wherein
figs. 1-3 shows in perspective views the sequences of use for a first embodiment of a drug delivery device,
fig. 4 shows in a non-assembled state a needle unit and a reservoir unit for a further embodiment of a drug delivery device,
fig. 5 shows an exploded view of the needle unit of fig. 4,
fig. 6 shows a perspective view of the needle unit of fig. 4 in a first state,
fig. 7 shows a perspective view of the needle carrier of fig. 5,
fig. 8 shows a perspective view of the needle unit of fig. 4 in a second state,
fig. 9 shows a side view of the needle unit of fig. 4,
fig. 10 shows a further perspective view of the needle unit of fig. 4,
fig. 11 shows perspective view of the interior of the reservoir unit of fig. 4,
fig. 12 shows an exploded view of a further reservoir unit,
figs. 13A and 13B show in a schematic representation a transcutaneous device in the form of a cannula and insertion needle combination,
fig. 14 shows a side view of a medical device mounted on a curved skin surface,
figs. 15A-15G shows different embodiments of a double or semi-double lumen cannula in combination with an insertion needle,
figs. 16A and 16B show two versions of a multi-material catheter,
figs. 16C-16E show different stages of use for a multi-material catheter,
figs. 17A and 17C show two embodiments of a flexible mechanical insertion needle arranged in a cannula,
fig. 17B shows a cross-sectional view of the insertion needle of fig. 17A,
fig. 17D shows a per se well known type of toy employing the principle shown in fig. 17A,
figs. 18A-18C show a bend insertion needle providing low height for insertion of a soft catheter,
figs. 19A-19C show an alternative version of the embodiment of figs. 18A-18C,
figs. 20A-20C show a medical device with a collapsible needle,
fig. 21A shows a U-shaped spring-steel needle in combination with a soft catheter,
figs. 21B and 21C show cross-sectional views of the embodiment of fig. 21A,
fig. 21D shows a tape measure embodying the principle of the needle of fig. 21A,
figs. 22A-22C show a device with an arcuately bend needle for insertion of a soft catheter,
fig. 23A shows a fibre needle for insertion of a soft catheter,
figs. 23B and 23C show cross-sectional views of two embodiments of a fibre needle,
figs. 24A-24D show a skin-mountable device in which a main spring loads two secondary springs for insertion of a cannula,
figs. 25A-25D show a skin-mountable device embodying a multi step concept with a single spring for insertion and retraction of a soft catheter,
figs. 26A-26D show a disposable inserter for insertion of a cannula in a medical device,
figs. 27A-27D show an integrated collapsible inserter for insertion of a cannula in a medical device, and
figs. 28A-28G show a disposable inserter in combination with a compact soft patch pump assembly.

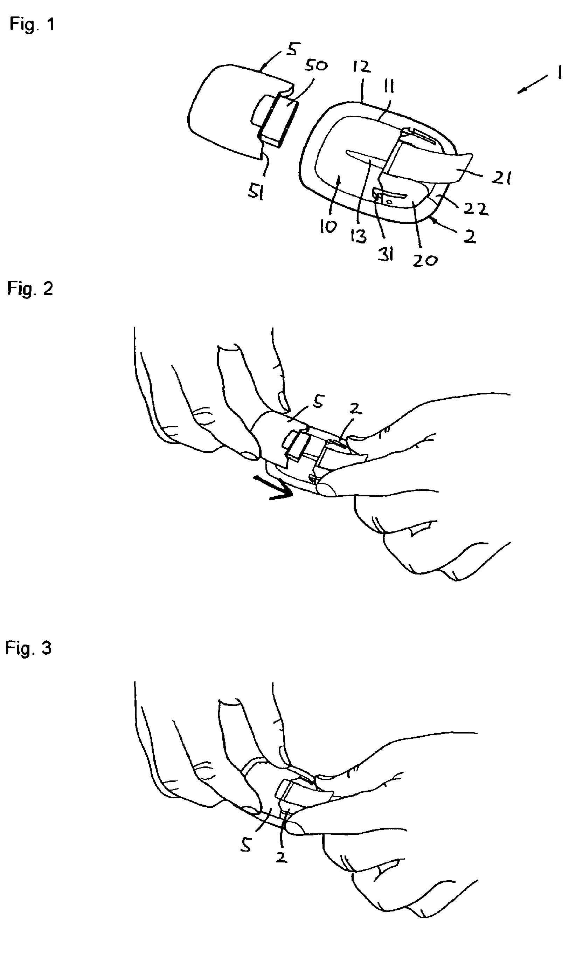

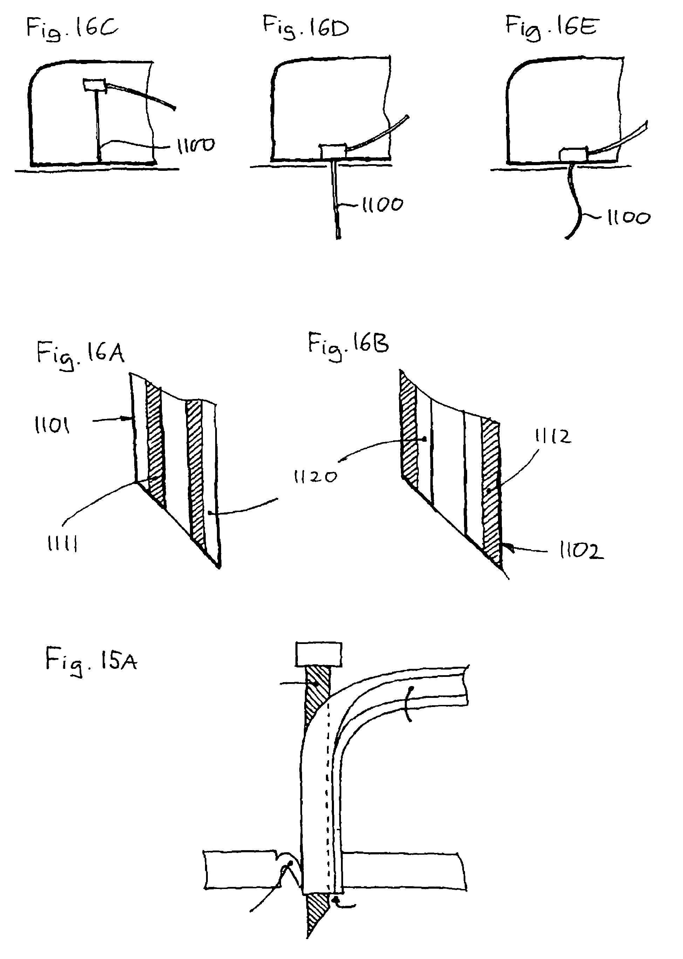

In the figures like structures are mainly identified by like reference numerals. Figs. 15A-28G does not show a medical device comprising a transcutaneous device unit and a process unit, but the described concepts for the introduction of a cannula can be used in combination with a ventilated skin mountable device as described with reference to figs 1-14.

### DESCRIPTION OF EXEMPLARY EMBODIMENTS

When in the following terms such as "upper" and "lower', "right" and "left", "horizontal" and "vertical" or similar relative expressions are used, these only refer to the appended figures and not to an actual situation of use. The shown figures are schematic representations for which reason the configuration of the different structures as well as there relative dimensions are intended to serve illustrative purposes only.

Firstly, with reference to figs. 1-3 an embodiment of a medical device for drug delivery will be described focusing primarily on the directly user-oriented features. The transcutaneous device unit 2 comprises a transcutaneous device in the form of a hollow infusion device, e.g. a needle or soft cannula, and will thus in the following be termed a needle unit, however, the needle may be replaced with any desirable transcutaneous device suitable for delivery of a fluid drug or for sensing a body parameter.

More specifically, fig. 1 shows a perspective view of medical device in the form of a modular skin-mountable drug delivery device 1 comprising a patch-like needle unit 2 and a reservoir unit 5. When supplied to the user each of the units are preferably enclosed in its own sealed package (not shown).

The needle unit comprises a flexible patch portion 10 with a lower adhesive mounting surface adapted for application to the skin of a user, and a housing portion 20 in which a hollow infusion needle (not shown) is arranged. The needle comprises a skin-penetrating distal end, e.g. pointed, adapted to penetrate the skin of a user, and is adapted to be arranged in fluid communication with the reservoir unit. In the shown embodiment the pointed end of the needle is moveable between an initial position in which the pointed end is retracted relative to the mounting surface, and an extended position in which the pointed end projects relative to the mounting surface. Further, the needle is moveable between the extended position in which the pointed end projects relative to the mounting surface, and a retracted position in which the pointed end is retracted relative to the mounting surface. The needle unit further comprises user-gripable actuation means in the form of a first strip-member 21 for moving the pointed end of the needle between the initial and the second position when the actuation means is actuated, and user-gripable retraction in the form of a second strip-member 22 means for moving the pointed end of the needle between the extended and the retracted position when the retraction means is actuated. As can be seen, the second strip is initially covered by the first strip. The housing further comprises user-actuatable male coupling means 31 in the form of a pair of resiliently arranged hook members adapted to cooperate with corresponding female coupling means on the reservoir unit, this allowing the reservoir unit to be releasable secured to the needle unit in the situation of use. The flexible patch portion comprises a flexible sheet 12 and a flexible support plate 11 extending from the housing, the support plate further comprising a flexible ridge formed support member 13 extending from the housing. The support plate as well as the housing may be fully or partly attached to the flexible sheet, e.g. by welding or adhesives. In case the housing and/or plate are only partly attached (i.e. corresponding to one or more areas located between the sheet and the housing and/or plate) the sheet will be able to partially move relative to the housing and/or plate. In use a peripheral portion of the sheet extends from the assembled device as the reservoir unit covers only the support plate of the upper surface of the patch. The adhesive surface is supplied to the user with a peelable protective sheet.

The reservoir unit 5 comprises a pre-filled reservoir containing a liquid drug formulation (e.g. insulin) and an expelling assembly for expelling the drug from the reservoir through the needle in a situation of use. The reservoir unit has a generally flat lower surface adapted to be mounted onto the upper surface of the patch portion, and comprises a protruding portion 50 adapted to be received in a corresponding cavity of the housing portion 20 as well as female coupling means 51 adapted to engage the corresponding hook members 31 on the needle unit. The protruding portion provides the interface between the two units and comprises a pump outlet and contact means (not shown) allowing the pump to be started as the two units are assembled. The lower surface also comprises a window (not to be seen) allowing the user to visually control the contents of the reservoir before the two units are connected.

First step in the mounting procedure is to assemble the two units by simply sliding the reservoir unit into engagement with the needle unit (fig. 2). When the hook members properly engage the reservoir unit a "click" sound is heard (fig. 3) signalling to the user that the two units have been properly assembled. If desired, a visual or audible signal may also be generated. Thereafter the user removes the peelable sheet 14 to uncover the adhesive surface where after the device can be attached to a skin surface of the user, typically the abdomen infusion of drug is started by gripping and pulling away the actuation strip 21 as indicated by the arrow whereby the needle is inserted followed by automatic start of the infusion. The needle insertion mechanism may be supplied in a pre-stressed state and subsequently released by the actuation means or the needle insertion may be "energized" by the user. A "beep" signal confirms that the device is operating and drug is infused. The reservoir unit is preferably provided with signal means and detection means providing the user with an audible alarm signal in case of e.g. occlusion, pump failure or end of content.

After the device has been left in place for the recommended period of time for use of the needle unit (e.g. 48 hours) - or in case the reservoir runs empty or for other reasons - it is removed from the skin by gripping and pulling the retraction strip 22 which leads to retraction of the needle followed by automatic stop of drug infusion where after the strip which is attached to the adhesive patch is used to remove the device from the skin surface.

When the device has been removed the two units are disengaged by simultaneously depressing the two hook members 31 allowing the reservoir unit 5 to be pulled out of engagement with the needle unit 2 which can then be discarded. Thereafter the reservoir unit can be used again with fresh needle units until it has been emptied.

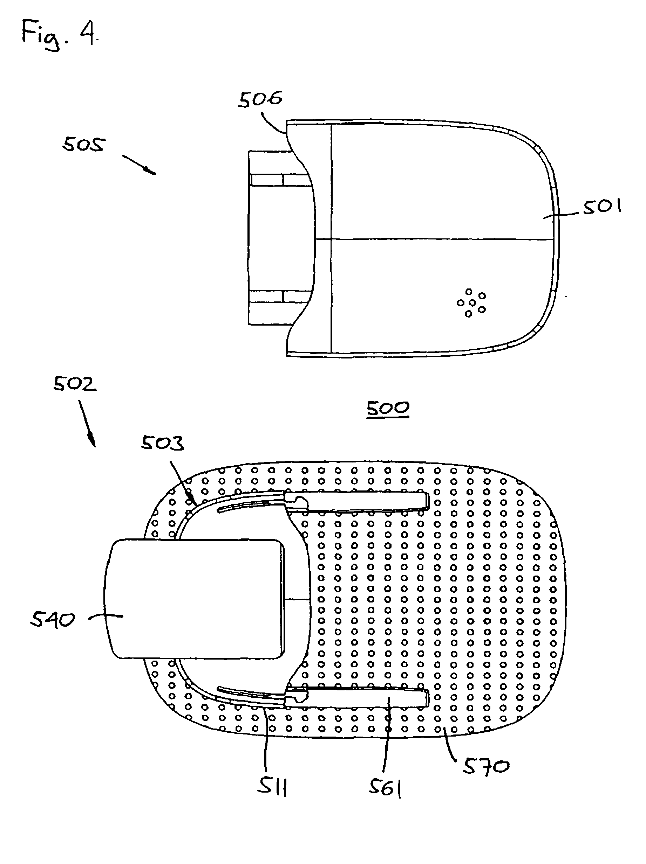

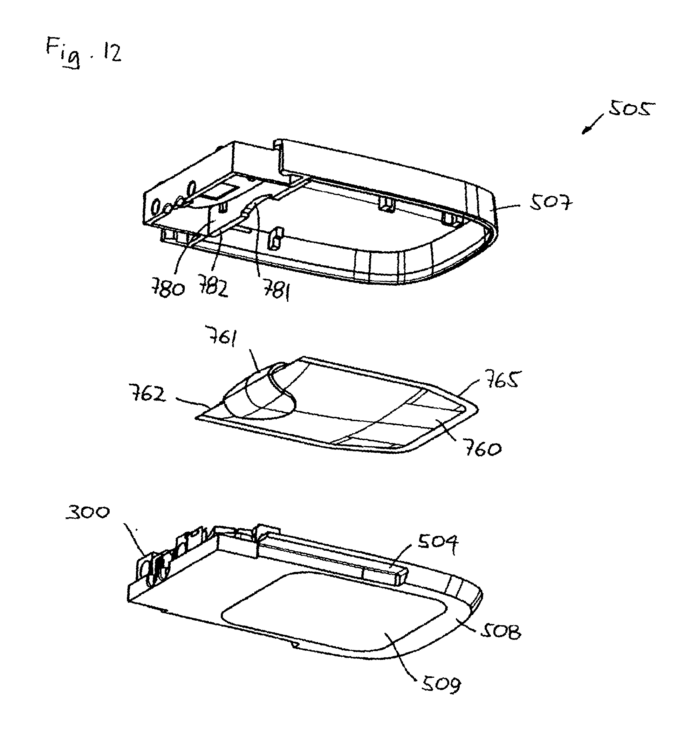

Fig. 4 shows a further embodiment of medical device 500 substantially corresponding to the embodiment of fig.1, the device comprising a transcutaneous device unit 502 and a process unit 505, More specifically, the transcutaneous device unit comprises a flexible patch portion (in the shown embodiment formed by a perforated sheet member 570) comprising an upper surface and a lower surface, the lower surface being adapted for application to the skin of a subject, a first housing 503 comprising a first coupling with two male coupling elements 511, and a transcutaneous device arranged in the housing (see below). In contrast to the embodiment of fig. 1, this embodiment does not comprise a support plate but instead two supporting ridge members 561 extend from the first housing and are attached to the upper surface of the sheet member. The supports serve as attachment supports for the first housing, however, they may also serve to control the distance between the lower surface or the process unit and the patch. When the second unit is configured to accommodate at least partially the support members, e.g. in corresponding cut-out portions or grooves 504 (see fig. 12), the supports may also serve to laterally stabilize the connection between the two units. The process unit comprises a second housing 501 with a lower surface and a second coupling arranged at a peripheral portion of the second housing, and a process assembly, e.g. a pump assembly as will be described below. In the shown embodiment the process unit has a generally flat rectangular shape with a cut-off end portion defining the interface with the transcutaneous device unit and also comprising the coupling in the form of two female coupling elements 506 arranged at each side of the end portion. Corresponding to figs. 1-3, the first and second couplings can be connected to each other with the upper surface of the patch facing towards the lower surface of the second housing. Due to the peripheral arrangement of the second coupling the flexible patch portion facing towards the lower surface of the second housing is free to move relative thereto, the degree of freedom being determined by the flexibility of the patch and supports if so provided and, of course, the surface to which the transcutaneous device unit is mounted.

In the shown embodiment the patch portion has the same general shape as the combined device albeit somewhat larger. In alternative embodiments the patch may comprise openings or cut-out portions. For example, an area between the two support legs may be cut out allowing the underlying skin to better breath. In the shown embodiments the couplings are arranged on "vertical" portions of the housings, however, coupling components may also be arranged on "horizontal" portions of the housings.

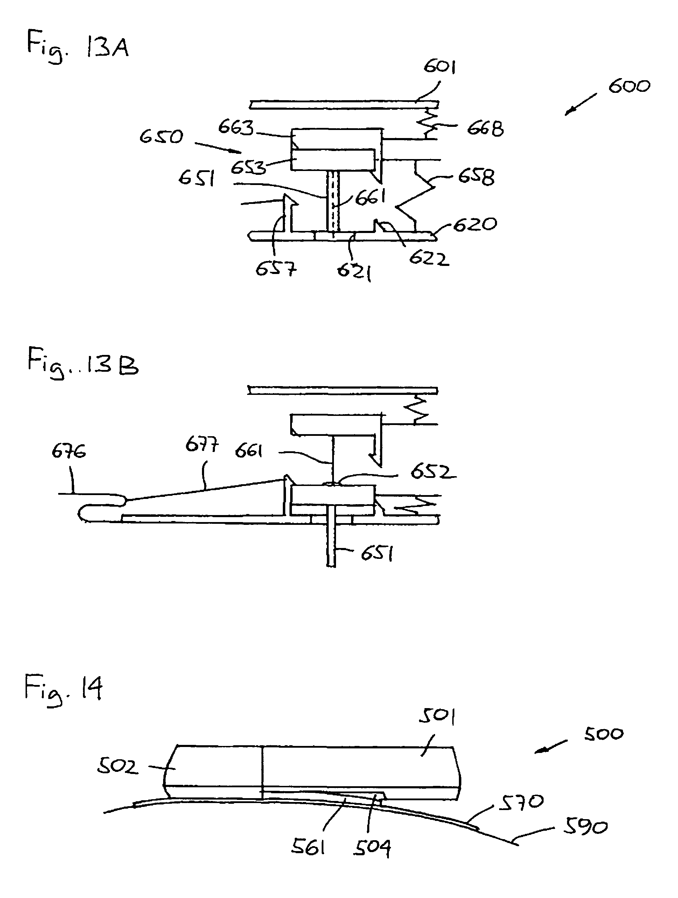

Fig. 14 shows a side view of the assembled device 500 mounted on a curving skin surface 590. As appears, the flexible patch portion with its support members is allowed to follow the curvature of the skin, this creating a ventilation space between the process unit and the patch portion. In case the needle housing is not fully attached to the flexible sheet, then a portion of the sheet located below the needle housing would also be allowed to follow the curvature of the skin and thus provide a ventilation space between the needle unit and the patch portion. In case no flexible patch portion is arranged below a portion of the process unit, i.e. the lower surface of the process unit is facing, directly towards the skin, then that portion of the process unit is indeed allowed to move relative to the skin.

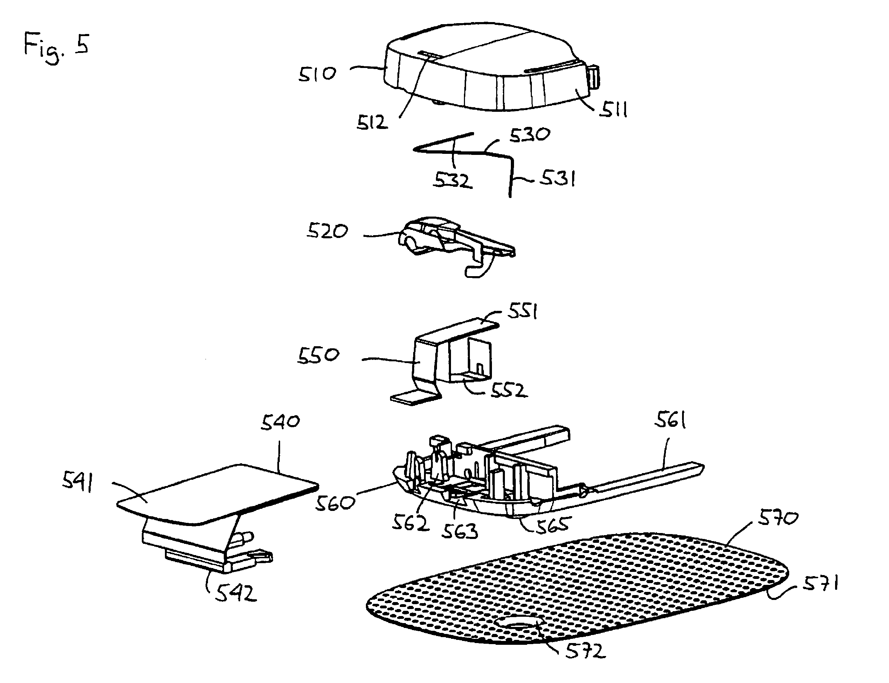

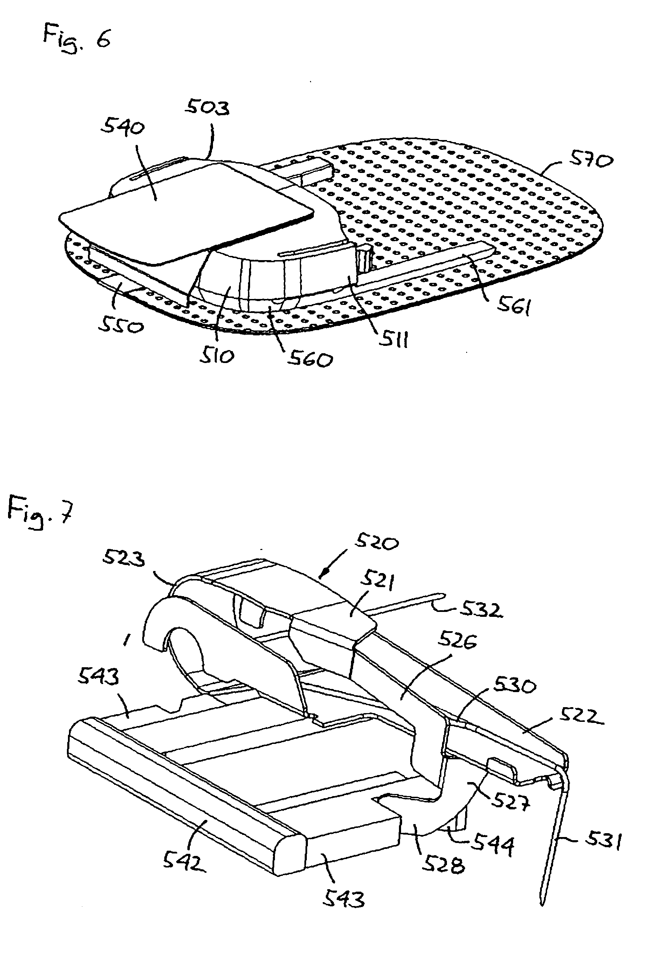

Fig. 5 shows an exploded perspective view of the needle unit comprising an upper housing portion 510, a needle carrier 520 and a thereto mounted infusion needle 530, an actuation member 540, a release member 550, a lower housing portion 560 and a sheet member 570. The actuation member comprises a user gripable portion 541 and a needle actuation portion 542, and the release member comprises a user gripable portion 551 and a needle retraction portion 552. In the assembled state as shown in fig. 6, the upper and lower housing portions form a housing 503 in which the needle and the needle carrier is mounted, the actuation and release members being operatable connected to the needle carrier with the user gripable portions arranged outside the housing. The sheet member further comprises an opening 572 arranged in register with a lower protrusion 565 provided around the exit aperture for the transcutaneous device, just as the sheet is provided with a large number of small perforations to improve breathability through the sheet. The housing 503 is provided with user actuatable coupling means 511 allowing a reservoir unit to be attached to and released from the needle unit 505, the reservoir unit comprising corresponding mating coupling means 506 as well as a display 587. The display may indicate e.g. proper function of the unit, the amount of drug in the reservoir or different error conditions.

As seen is the user gripable portion 551 of the release member initially covered by a portion of the actuation member, this reducing the probability that the user erroneously uses the release member instead of the actuation member. Further, the actuation and release members (or portion thereof) may be colour coded to further assist the user to correctly use the device. For example, the actuation member may be green to indicate "start" whereas the release member may be red to indicate "stop".

Fig. 7 shows in perspective the needle carrier 520 with the needle 530 and the needle actuation portion 542 of the actuation member 540. The needle actuation portion comprises two legs 543 allowing it to slide relative to the housing, the legs being arranged through respective openings 563 in the housing. The needle carrier is adapted to be connected to a hinge member 562 of the lower housing portion to thereby allow the needle carrier and thereby the needle to pivot corresponding to a pivoting axis defined by a hinge. In the shown embodiment is the needle carrier in the form a bent sheet metal member, the carrier comprising an upper arm 521 and a lower arm 522 connected to each other by a hinge portion 523 allowing the lower arm to pivot relative to the upper arm and corresponding to the pivoting axis. The lower arm forms a tray in which the hollow infusion needle 530 is mounted (e.g. by welding or adhesive), the needle having a distal pointed portion 531 adapted to penetrate the skin of the subject, the distal portion extending generally perpendicular to the mounting surface of the needle unit, and a proximal portion 532 arranged substantially corresponding to the pivoting axis and adapted to engage a fluid supply. Thus, when a portion of the upper arm is mounted in the housing, the lower arm can pivot between a first retracted position in which the distal portion of the needle is retracted within the housing, and a second extended position in which the distal portion projects relative to the mounting surface. In the shown embodiment the needle carrier provides the drive means for moving the lower arm between the two positions. This may as in the present embodiment be provided by the elastic properties of the sheet material per se corresponding to the hinge portion, or alternatively an additional spring may be provided between the two arms to thereby urge them apart. To lock the lower part in an energized, releasable first position, the upper arm is provided with a flexible release arm 526 comprising a catch 527 supporting and arresting the lower arm in its first downwardly biased position, as well as a release portion 528 engaging a ramp surface 544 of the needle actuation portion 542, the catch further comprising an inclined edge portion 529 adapted to engage the lower arm when the latter is moved from its extended to its retracted position as will be described in greater detail below.

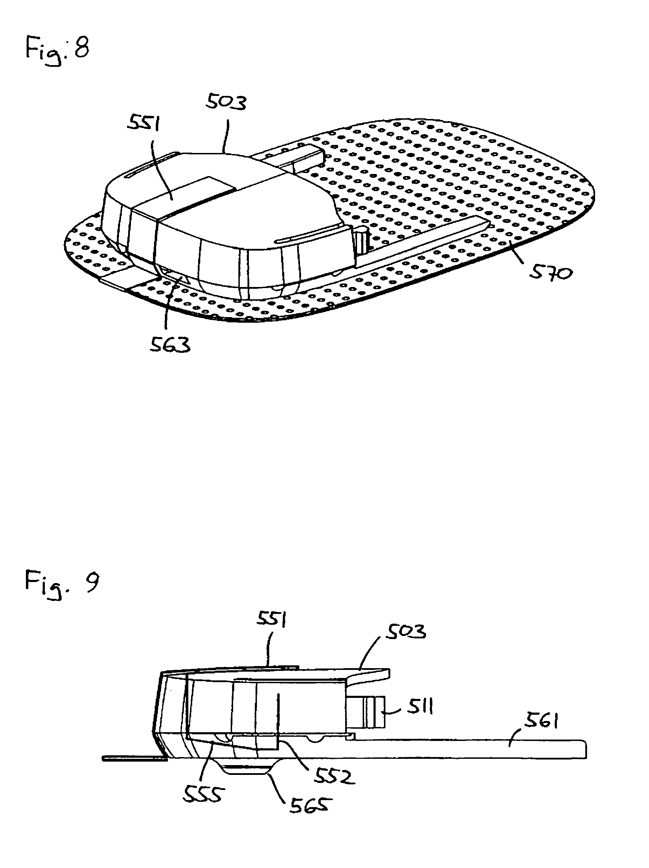

To actuate the needle the user grips the flexible strip forming the user gripable portion 541 (which preferably comprises adhesive portions to hold it in its shown folded initial position) and pulls the needle actuation portion 542 out of the housing, the actuation member 540 thereby fully disengaging the housing. More specifically, when the ramp surface 544 is moved it forces the latch 527 away from the lower arm to thereby release it, after which the release portion 528 disengages the ramp allowing the two legs to be pulled out of the housing. As seen in fig. 8, when the actuation member is removed the user gripable portion 551 of the release member is exposed. As for the actuation member, the user gripable portion of the release member preferably comprises adhesive portions to hold it in its shown folded initial position.

In the shown embodiment the release member is in the form of a strip formed from a flexible material and having an inner and an outer end, the strip being threaded through an opening 512 in the housing, the strip thereby forming the user gripable portion 551 and the needle retraction portion 552, the inner end of the strip being attached to the housing and the outer end of the strip being attached to a peripheral portion of the sheet member 570 or, alternatively, a peripheral portion of the housing. In the projection shown in fig. 9 the release member is shown in its initial position, the retraction portion forming a loop 555 arranged below the lower arm of the needle carrier, this position allowing the lower arm to be moved to its actuated position and thereby the needle to its extended position.

When the user decides to remove the needle unit from the skin, the user grips the user gripable portion 551, lifts it away from the housing and pulls it upwardly whereby the loop shortens thereby forcing the lower arm upwardly, this position corresponding to an intermediate release state. By this action the lower arm engages the inclined edge portion 529 of the catch 527 thereby forcing it outwardly until it snaps back under the lower arm corresponding to the position shown in fig. 7. As the actuation member 540 has been removed from the needle unit, the needle carrier is irreversibly locked in its retracted position. When the user further pulls in the release member, the peripheral portion of the sheet member to which the release member is attached will be lifted off the skin, whereby the needle unit with its attached reservoir unit can be removed from the skin, this as described above.

Advantageously, the actuation and release members may be formed and arranged to communicate with the reservoir unit (not shown). For example, one of the legs of the actuation member may in its initial position protrude through the housing to thereby engage a corresponding contact on the reservoir unit, this indicating to the reservoir unit that the needle unit has been attached, whereas removal of the actuation member will indicate that the needle has been inserted and thus that drug infusion can be started. Correspondingly, actuation of the release member can be used to stop the pump.

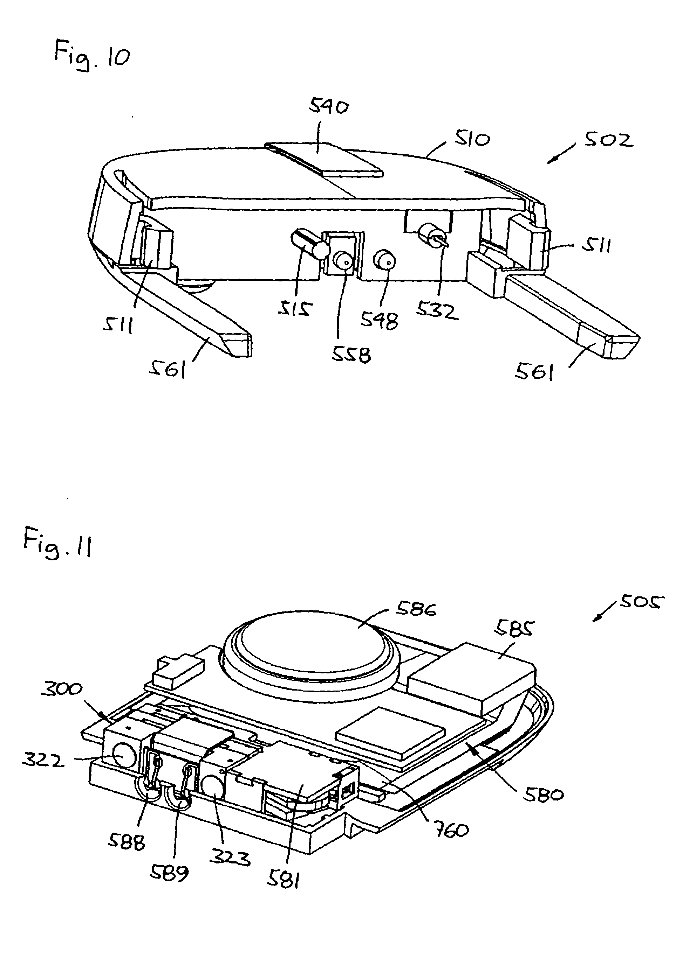

In fig. 10 the side of the needle unit 502 which connects to the reservoir unit is shown. In addition to the two ridge members 561 and the user actuatable coupling means 511 the needle unit comprises further structures which connects to and/or engages the reservoir unit to provide a functional interface with the reservoir unit. More specifically, the needle unit comprises a fluid inlet provided by the pointed proximal portion 532 of the needle projecting from the needle unit and adapted to engage a fluid outlet of the reservoir unit, an actuator 515 projecting from the needle unit and adapted to engage and actuate a fluid connector in the reservoir unit (see below), and first and second contact actuators 548, 558 adapted to engage corresponding contacts on the reservoir unit. The first contact actuator is provided by the distal end of one of the legs 543 of the needle actuator projecting through an opening in the housing, and the second contact actuator is provided by a hinged portion of the housing connected to the needle retraction portion 552 of the release member 550. When the needle unit is first connected to the reservoir unit both contact actuators will protrude from the housing and engage the corresponding contacts on the reservoir unit thereby indicating that that a needle unit has been connected. When the needle is actuated the first contact actuator will be withdrawn and thereby disengage the corresponding contact on the reservoir unit to start pump actuation. When the needle is retracted the second contact actuator will pivot and disengage the corresponding contact on the reservoir unit to stop pump actuation.

Fig. 11 shows the reservoir unit with an upper portion of the housing removed. The reservoir unit comprises a reservoir 760 and an expelling assembly comprising a pump assembly 300 and control and actuation means 580, 581 therefore. The pump assembly comprises an outlet 322 for connection to a transcutaneous access device (e.g. the needle 530) and an opening 323 allowing an internal fluid connector to be actuated, see below. The reservoir 560 is in the form of prefilled, flexible and collapsible pouch comprising a needle-penetratable septum adapted to be arranged in fluid communication with the pump assembly, see below. The shown pump assembly is a mechanically actuated membrane pump, however, the reservoir and expelling means may be of any suitable configuration.

The control and actuation means comprises a pump actuating member in the form of a coil actuator 581 arranged to actuate a piston of the membrane pump, a PCB or flex-print to which are connected a microprocessor 583 for controlling, among other, the pump actuation, contacts 588, 589 cooperating with the contact actuators on the needle unit, signal generating means 585 for generating an audible and/or tactile signal, a display (not shown) and an energy source 586. The contacts are preferably protected by membranes which may be formed by flexible portions of the housing.

In fig. 12 an exploded view of the reservoir unit 505 of fig. 4 is shown, the unit comprising an upper housing member 507, a lower housing member 508 with a transparent area 509 and grooves 504 to receive the ridge members 561 extending from the needle unit, a flexible reservoir 760 with a rounded edge portion 762 on which a septum member 761 is mounted, a pump assembly 300 with actuator and a circuit board (not shown) arranged above the reservoir and comprising electronic components for controlling actuation of the pump. The upper and lower housing members comprise reservoir mounting means in the form of opposed upper and lower ridge portions 780 (the lower not seen) adapted to engage and mount the reservoir in the housing. Each ridge portion comprises a central cut-out portion 781 adapted to engage the septum member on its opposed surfaces when the housing members are assemble thereby locking the reservoir in place within the housing. The degree of locking will be determined by the pressure exerted on the septum member, the elastic properties of the septum member and the friction between the ridge and the septum member. On each side of the cut-out portion the ridge portions comprise a straight portion 782 which may aid in mounting the reservoir in the housing. The straight portions may engage the initially prefilled reservoir to help lock it in place, however, as the reservoir is emptied and flattens this grip may lessen. In contrast, the engagement with the septum is adapted to properly hold the reservoir in place as the reservoir is emptied. The straight portions may also be adapted to pinch and fully flatten the reservoir thus serving as an additional mounting means. Additional mounting means (not shown) may engage and grip the reservoir at other locations, e.g. along the welded edges 765.

In the above described embodiments, the transcutaneous device has been in the form of a unitary needle device (e.g. an infusion needle as shown or a needle sensor (not shown)), however, the transcutaneous device may also be in the form of a cannula or a sensor in combination with an insertion needle which is withdrawn after insertion thereof. For example, the first needle portion may be in the form of a (relatively soft) infusion cannula (e.g. a Teflon ® cannula) and a there through arranged removable insertion needle. This type of cannula needle arrangement is well known from so-called infusion sets, such infusion sets typically being used to provide an infusion site in combination with (durable) infusion pumps.

Thus, figs. 13A and 13B show in a schematic representation how a cannula and insertion needle combination can be arranged within a housing 601 of in a given medical device 600 (partly shown), e.g. an infusion device or an infusion set. More specifically, the medical device comprises a transcutaneous assembly 650 comprising a combination of a relatively soft cannula 651 (which e.g. may be of the soft "Teflon®" type) carried by a lower member 653 and a pointed insertion needle 661 (e.g. made from medical grade stainless steel) slidably arranged within the cannula and carried by an upper member 663, both members being mounted to allow axial displacement of the cannula respectively the insertion needle. The cannula comprises a proximal inlet (not shown) allowing it to be or to be arranged in fluid communication with a fluid source. The medical device further comprises a base plate 620 with an opening 621 for the cannula as well as a release member 622. The lower member comprises an elastomeric seal 652 through which the insertion needle is arranged. The cannula and the insertion needle may be straight or curved dependent upon how the two members are mounted in the device, e.g. arcuate corresponding to a pivoting axis or straight corresponding to linear movement as illustrated. The upper member comprises a coupling member 667 locking the members together in an initial position with distal end of the insertion needle extending from the distal opening of the cannula as shown in fig. 13A, and the base plate comprises coupling member 657 for locking the lower member in an extended position with distal end of the cannula extending through the opening in the base plate (see fig. 13B). Between the housing of the device and the upper member a first spring 668 is arranged biasing the upper member upwards. Correspondingly, the device also comprises a second spring 658 biasing the lower member upwardly. The medical device further comprises a gripping tab 676 and a pulling member 677 corresponding to the embodiment shown in fig. 1.

In a situation of use the assembly is moved downwardly, either manually or by a releasable insertion aid, e.g. a spring loaded member acting through an opening in the housing (not shown) whereby the cannula with the projecting insertion needle is inserted through the skin of a subject. In this position the lower member engages the coupling member 657 to thereby lock the cannula in its extended position, just as the coupling member 667 is released by the release member 622 thereby allowing the upper member to return to its initial position by means of the first spring.

When the user intends to remove the delivery device from the skin surface, the user grips the gripping portion of the tab and pulls it in a first direction substantially in parallel with the skin surface, by which action the flexible strip 677 releases the coupling member 657 from the lower member whereby the lower member and thereby the cannula is retracted by means of the second spring. When the cannula has been withdrawn from the skin, the user uses the now unfolded tab to pull off the entire delivery device from the skin surface, for example by pulling the tab in a direction away from the skin surface.

In the above description of the preferred embodiments, the different structures and means providing the described functionality for the different components have been described to a degree to which the concept of the present invention will be apparent to the skilled reader. The detailed construction and specification for the different components are considered the object of a normal design procedure performed by the skilled person along the lines set out in the present specification.

In the following a number of concepts for the introduction of a cannula, e.g. a soft cannula or catheter, will be described. Advantageously, the different concepts may be used in combination with a ventilated skin mountable device as described above.

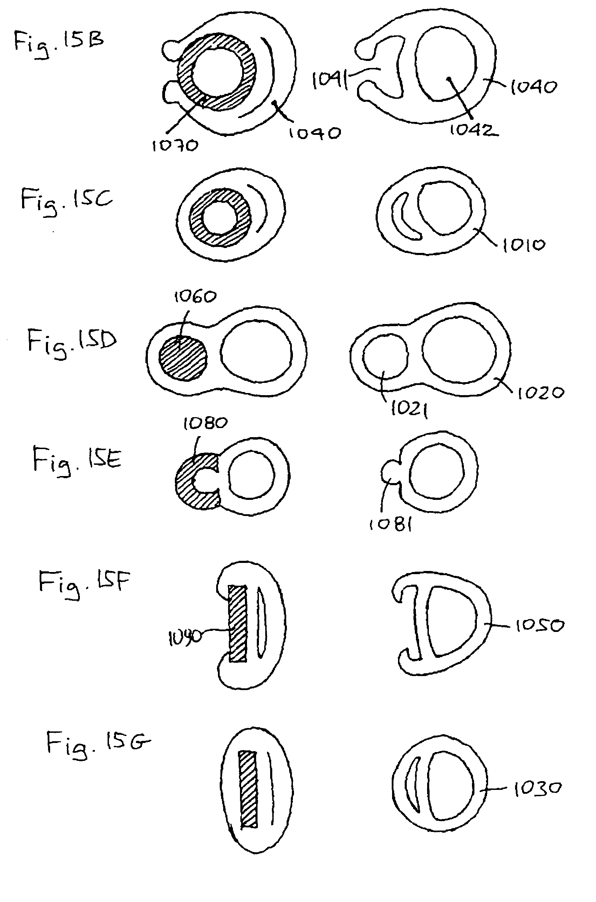

Figs. 15A-15G show an arrangement utilizing double and semi-double lumen Teflon ® tubing for insertion of a soft catheter for subcutaneous drug delivery. As shown in figs. 15C, 15D and 15G the concept utilizes (i) a double lumen catheter 1010, 1020, 1030, or (ii) a semi-double lumen catheter 1040, 1050 (as shown in figs. 15B and 15F), wherein a solid or hollow needle 1060, 1070, 1090 is positioned in one of the lumens 1021 or semi-lumens 1041 and insulin is distributed by the other lumen. Alternatively, as shown in fig. 15E, a needle may be attached laterally. In this way the needle can be kept separate, so that needle can be removed from catheter without influencing the insulin distribution lumen. Insulin distribution lumen can be closed while needle is positioned in other lumen. Accordingly, a cannula insertion device is provided comprising a flexible cannula and a needle with a pointed distal end, the cannula comprising a conduit with a distal end opening and along a length thereof from the distal opening a holding structure for holding the needle in parallel with a distal portion of the cannula, the pointed end of the needle projecting relative to the distal end opening. The cannula and needle are moveable from an initial position arranged within the device to an extended position projecting from the device. When the cannula and needle has been inserted the needle can be withdrawn from the cannula. In an exemplary embodiment the cannula 1010, 1030, 1040, 1050 along the needle is compressed, this providing a smaller cross-section during insertion. When the needle is withdrawn the flexible conduit expands to reestablish the conduit space 1042 within the initially compressed cannula. The holding structure may fully or partly encircle the needle just as the needle 1080 may be formed to better engage a given holding structure 1081. An advantage of this concept is the low height of device housing needed to keep needle and soft catheter within device, without removing needle totally when pulled out of soft catheter. A further advantage is that a double lumen soft catheter can be quite compact when inserted, as insulin distribution lumen can be closed while needle is positioned in other lumen.

Figs. 16A and 16B show two versions of a multi-material biological catheter for insertion of a soft catheter 1100 for subcutaneous drug delivery. The concept utilizes a special extruded soft catheter 1101, 1102, with two or more different materials, the multi-material catheter walls are completely stiff and strong with a sharp cutting edge before insertion, without a need of assisting needle for insertion. When inserted the influence from the body-temperature/humidity or influence from insulin makes the combination of materials soft within the body. One material could even be a bio-plastic which is dissolved by the influence of the body or insulin. Such materials are generally known, e.g. thread used for operations which dissolves in the body after a period. Accordingly, a cannula is provided comprising a conduit with a distal opening, and being formed from a flexible durable material, the cannula further comprising a support formed along the distal portion of the conduit, the support being formed from a material having an initial relative rigid state providing a support for the cannula during insertion through the skin of a subject, and a second relatively flexible state when subjected to body conditions or a drug as indicated above. The support 1111, 1112 may be arranged on the external and/or internal surface of the cannula 1120, just as it may fully or partly support the cannula around the full circumference. An advantage of this invention is that multi-material catheter is stiff before insertion and gets soft by influence of the body or insulin after insertion (see figs. 16C-16E). A further advantage is that there is no need of a needle for insertion and therefore no need of removing needle after insertion.

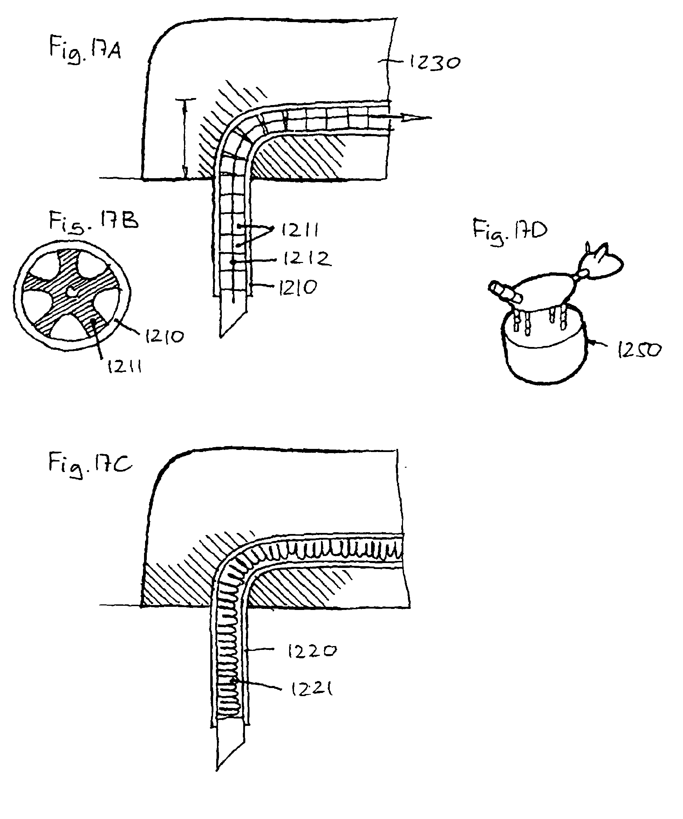

Figs. 17A and 17C show a flexible mechanical insertion needle for insertion of a soft catheter for subcutaneous drug delivery. The flexible mechanical insertion needle 1210, 1220 comprises (a) a plurality of links 1211 on a wire 1212, or (b) a guide wire 1221. In respect of (a), when the Wire is tightened the needle is rigid, but when the wire is loosened the needle becomes flexible. This mechanism in also well known from the "flexible legs" type of toy 1250. Accordingly, a cannula insertion device 1230 is provided comprising a flexible cannula and a needle with a pointed distal end arranged there within, the needle being transformable between a first rigid state and a second relaxed state, this providing a support during insertion yet allows the cannula to flex when inserted. As the needle is left in the cannula after insertion, the needle has a cross-sectional configuration providing a fluid conduit along therewith. Thus, when the needle is rigid it assists the soft catheter during insertion, however, when the inserted wire is loosened the needle gets soft and stays within catheter and secures that catheter can not kink. In respect of (b), the guide wire assists insertion, but is soft after insertion and secures that catheter can not kink. An advantage of this concept is no need of removing insertion needle after insertion, as it can change character from rigid to soft. A further advantage is that the insertion needle is providing kink resistance to the soft catheter.

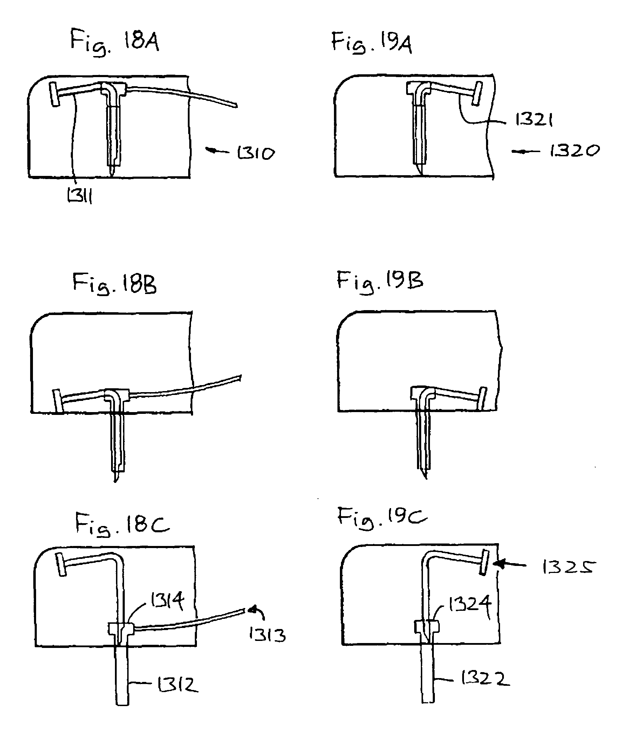

Figs. 18A-18C and 19A-19C show a bend needle providing low height for insertion of a soft catheter for subcutaneous drug delivery. The figures show two versions 1310, 1320 of a drug delivery device with catheters 1312, 1322. In figs. 18A-18C the needle 1311 is bent and placed in a notch in the catheter mounting and drug is distributed through a separate tube 1313. The needle is arranged such that it can stay in the proximal septum 1314 of the catheter when pulled proximally after insertion. The arrangement secures low height and allows the flow of drug through the catheter mounting. In figs. 19-19C the hollow needle 1321 is bent and placed through a septum 1324 in the catheter mounting and drug is distributed through the cannula via the needle proximal end 1325. Needle tip stays in septum and catheter mounting when pulled proximally after insertion. An advantage of this concept is the low height of device housing needed to keep needle and soft catheter within the device, without removing needle totally when pulled out of the soft catheter. A further advantage is that the needle and cannula are an integrated part of the drug delivery system.

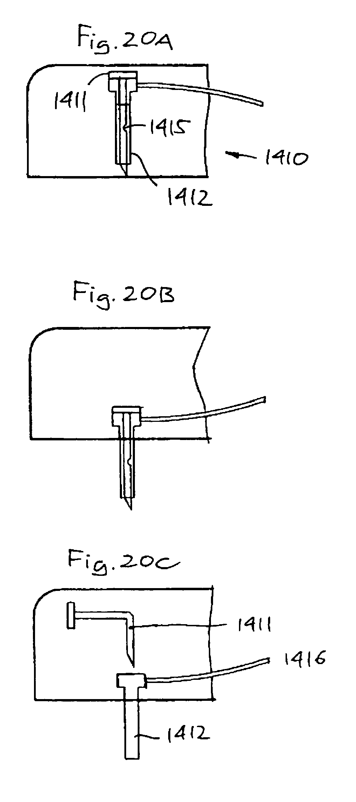

Figs. 20A-20C show a drug delivery device 1410 with a collapsible needle 1411 providing low pull-out height for insertion of a soft catheter 1412 for subcutaneous drug delivery. In accordance with the concept a soft catheter is inserted with a needle arranged there within. When the needle is pulled out after insertion it collapses or kinks to secure a low height of an enclosing device housing. Accordingly, a cannula insertion device is provided comprising a flexible cannula with a straight distal portion, and a needle with a pointed distal end arranged there within, the needle being transformable between a first generally straight configuration and a second bent (e.g. kinked or collapsed) configuration, this providing a straight support during insertion yet allows the cannula to be bent when withdrawn from the catheter. In the shown embodiment the needle has weak portion allowing the needle to kink at that point. Drug may be delivered to the cannula through a tubing 1416. An advantage of this concept is the low height of the housing cabinet needed to keep the needle and the soft catheter within the device.

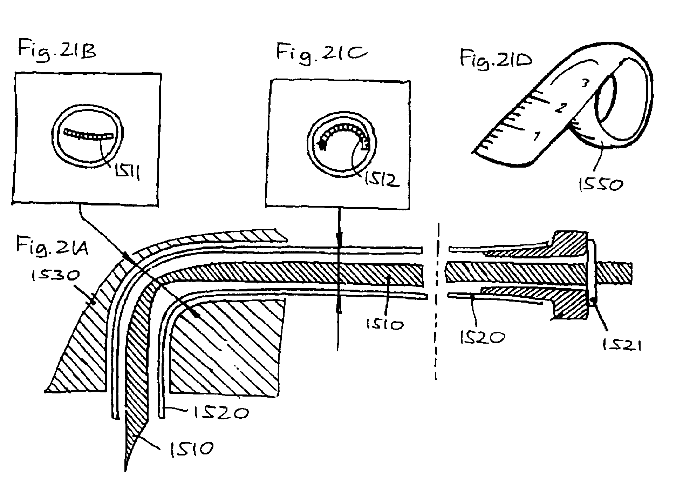

Fig. 21A shows a U-shaped spring-steel needle 1510 for insertion of a soft catheter 1520 for subcutaneous drug delivery. The soft catheter is inserted with a U-shaped spring-steel needle, wherein the shape of the guide adapts dynamically when the needle is forced against a guide 1530, this providing the guide with different cross-sections 1511, 1512. This allows the soft catheter with needle to be placed horizontally before vertical insertion, as a guide 1530 bends the catheter and needle during the insertion. U-shaped spring steel structures are generally known, e.g. from tape measures 1550. An advantage of this concept is the low height of a device housing needed to keep the needle and soft catheter within the device, without removing the needle fully when it after insertion is pulled proximally through the septum 1521. A further advantage is very little space occupied by the needle after insertion.

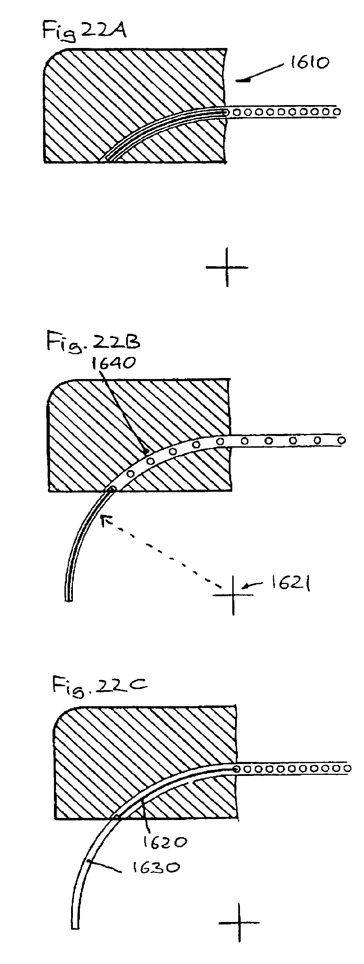

Figs. 22A-22C show a device 1610 with an arcuately bend needle 1620 for insertion of a soft catheter 1630 for subcutaneous drug delivery. The soft catheter is inserted with a moderately bend hollow needle 1620, e.g. arc shaped with a centre 1621, with a guide or channel 1640 securing correct arcuate insertion. The needle is moved proximally after insertion, but forms part of the fluid path for a drug delivery system, the needle being in sealed engagement with the catheter. The catheter can be expected to have a moderately arcuate form when inserted. An advantage of this concept is the low height of device housing needed to keep needle and soft catheter within the device, without removing needle fully when pulled out of soft catheter. A further advantage is that the needle is a part of the drug delivery path after insertion.

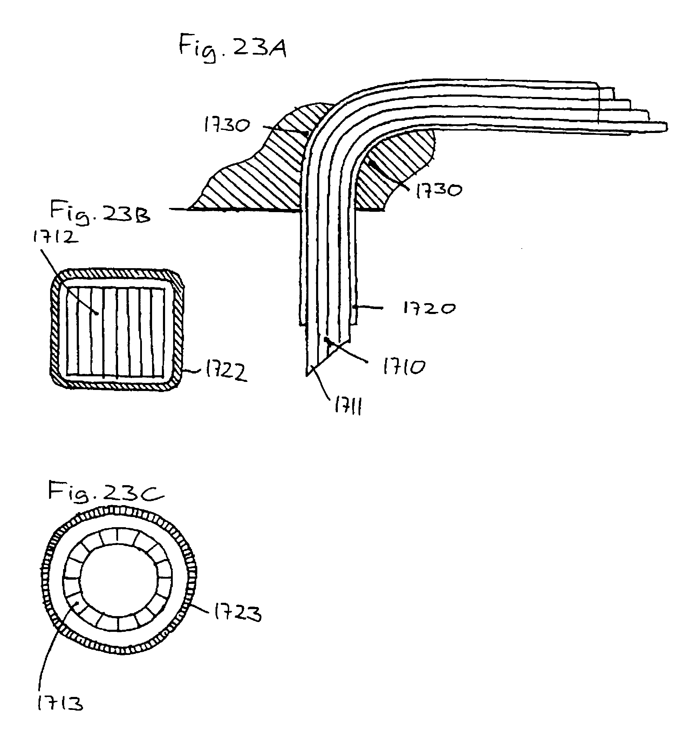

Fig. 23A shows a fibre needle 1710 for insertion of a soft catheter 1720 for subcutaneous drug delivery. A soft catheter is inserted with multiple layers 1711 or cords of fibres which are joined in distal end and directed/controlled at the proximal end. As the fibres are guided by the catheter, it is possible to completely control and direct the insertion of the fibre needle. In the shown embodiment the catheter is guided in a bend channel 1730. After insertion the fibre needle can be relaxed and thus flexible by stopping the control of the back end. As appears, this concept provides a specific solution to the principle discussed above in relation to figs. 17A-17C. As shown in fig. 23B, the fibre needle 1712 may take up substantially the entire cannula 1721 during insertion and thereafter be withdrawn, or it may allow fluid transport along its length and thus be left in place after insertion. Alternatively, the fibre needle may form a tubular structure 1713. An advantage of this concept is the low height of a device housing needed to keep the fibre needle and soft catheter within the device without removing needle fully when pulled out of soft catheter. A further advantage is that a fibre needle can be fully flexible after insertion, and stay inside the soft catheter. A yet further advantage is that the insertion guide supports the soft catheter against kinking.

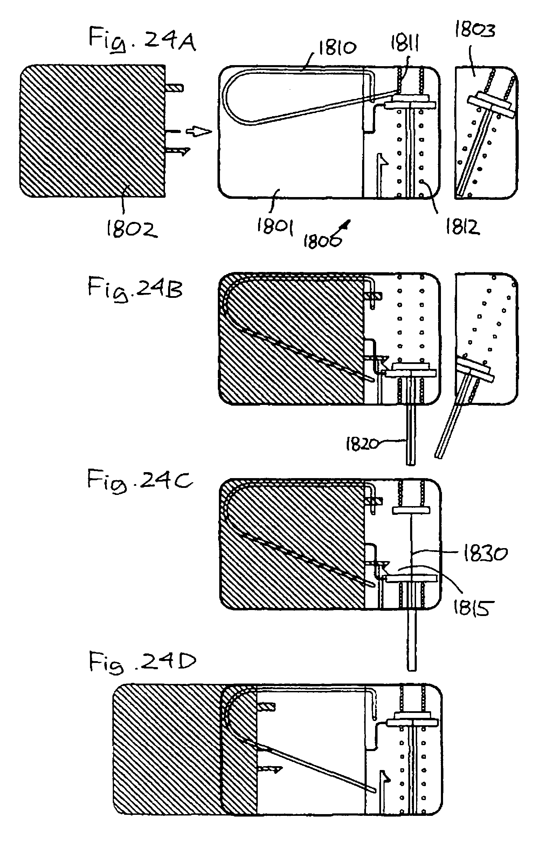

Figs. 24A-24D show a skin-mountable device 1800 comprising a soft catheter 1820 in which is arranged an insertion needle 1830. More specifically, the device comprises an arrangement in which one loaded main spring 1810 loads two secondary springs 1811, 1812 for insertion of the soft catheter 1820 for subcutaneous drug delivery. In the shown embodiment it is a pull-spring 1811 for the insertion needle, and for the soft catheter it is a push-spring 1822. The main spring is placed levelled on top of a patch sheet 1801. When e.g. a reservoir unit 1802 is connected it releases the loaded main spring that inserts the soft catheter with its needle. The insertion loads two springs for pull out of the needle respectively the soft catheter. The needle is pulled out by spring 1811 immediately after insertion of the soft catheter with its insertion needle, whereas the soft catheter is pulled out when the reservoir unit is removed from the skin-mountable device, this actuating the a release mechanism 1815. Instead of the "vertical" arrangement of the catheter, the catheter may be arranged inclined (see unit 1803). Accordingly, a medical device is provided comprising a first actuated (or actuatable) spring, second and third non-actuated springs, a cannula and a needle with a skin-penetrating distal end arranged there within (see fig. 24A). When the first spring is released the combined cannula and needle is inserted, and the second and third spring is actuated (see fig. 24B). When the second spring is released the needle is withdrawn (see fig. 24C).

This action may be automatically coupled to detection of the cannula having reached its extended position. When the third spring is released the cannula is withdrawn (see fig. 24D). An advantage of this invention is that only one main spring is loaded, which loads two secondary springs which are unloaded before use. A further advantage is that soft catheter with its needle is hidden before use, and that needle and soft catheter are pulled out at end of use when the device is disposed off.

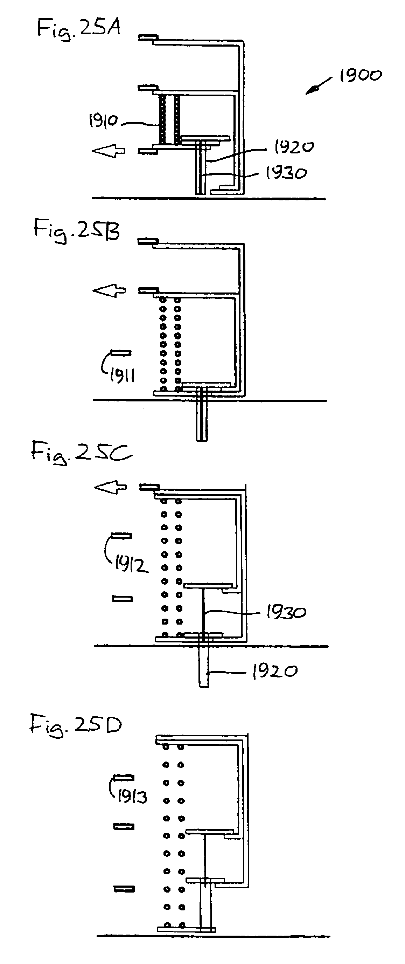

Figs. 25A-25D show a skin-mountable device 1900 comprising a soft catheter 1920 in which is arranged an insertion needle 1930, and embodying a multi step concept with a single loaded spring 1910 for insertion of a soft catheter for subcutaneous drug delivery. In accordance with this concept one single spring is loaded and has a multi step function: (a) The spring is loaded (alternatively the spring may be supplied pre-loaded) and a first locking structure 1911 is ready to be disengaged from the catheter assembly (see fig. 25A). (b) When the first locking structure is released the soft catheter with its insertion needle is inserted, and a second locking structure 1912 is ready to be disengaged (see fig. 25B). (c) When the second locking structure is disengaged the insertion needle is pulled proximally, and a third locking structure 1913 is ready to be disengaged (see fig. 25C). (d) When the third locking structure is disengaged the soft catheter is pulled out (see fig. 25D). The steps (a)-(c) or (b)-(c) may be combined and performed automatically when a user actuation means is actuated, e.g. as described above for the concept disclosed with reference to figs 24A-24D, the needle may be pulled out automatically when the cannula has reached its extended position. An advantage of this concept is that one spring is loaded, and provides all actuation for inserting and withdrawing the catheter/needle. A further advantage is that the soft catheter with the needle is hidden before use, and that the needle and soft catheter are pulled out at end of use when the device is disposed off.

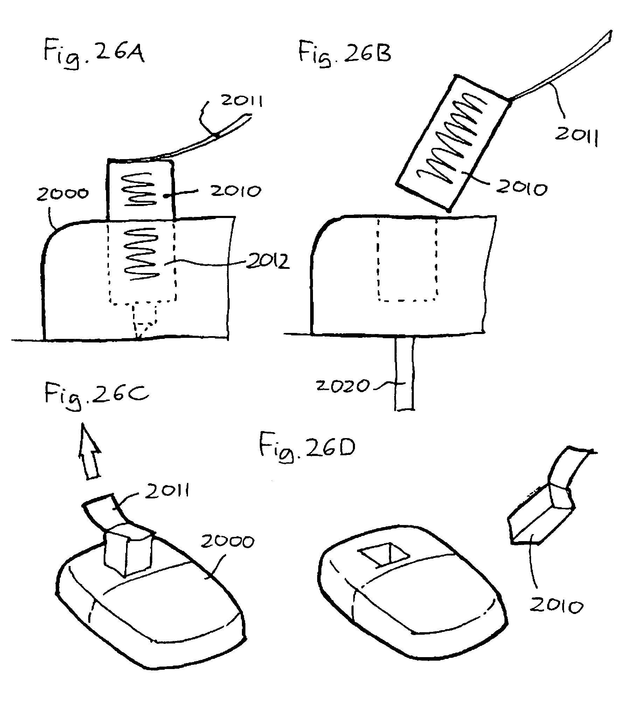

Figs. 26A-26D show a disposable inserter 2010 for insertion of a soft catheter 2020 for subcutaneous drug delivery. A device, e.g. a drug delivery device 2000, is supplied with a disposable inserter with a needle which is removed after insertion. A release strip 2011 is controlling insertion, pull out of needle as well as removal of the inserter after use. Accordingly, a medical device is provided comprising a housing portion to which a cannula can be attached in an extended position, and an inserter assembly comprising a flexible cannula and a needle with a pointed distal end arranged there within, the inserter being mountable to the medical device. When the inserter assembly is mounted to the medical device actuation thereof (e.g. by release of a pre-actuated spring 2012) moves the combined cannula and needle to an extended position in which the cannula engages the medical device. There after the inserter assembly with the needle can be removed from the medical device, the needle preferably being withdrawn there within. An advantage of this concept is that the complete inserter mechanism is pulled out of device and does not add weight and volume to the device in use. A further advantage is that the soft catheter with needle is hidden before use, and that soft catheter is pulled out at end of use and device is disposed off.

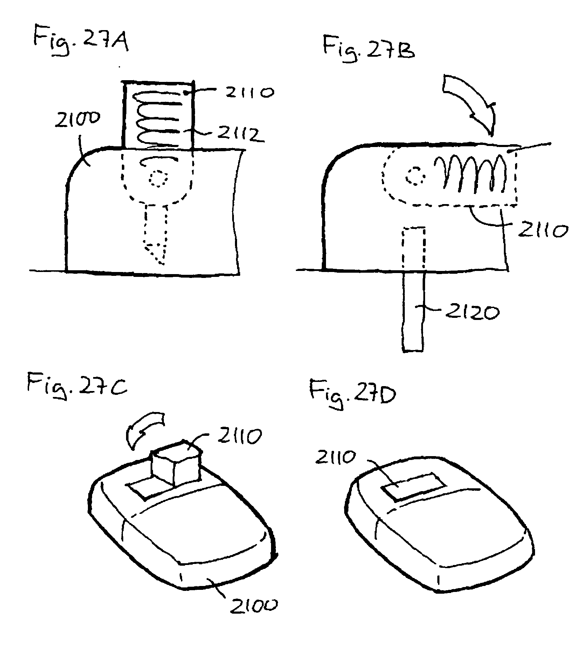

Figs. 27A-27D show an integrated collapsible inserter 2110 for insertion of a soft catheter 2120 for subcutaneous drug delivery. A device, e.g. a drug delivery device 2100, is supplied with a displaceable inserter assembly that is higher than the device before insertion but folds down and integrates fully or partly in an outer surface of the device after insertion. Accordingly, a medical device is provided comprising a housing portion to which a cannula can be attached in an extended position, and an inserter assembly comprising a flexible cannula and a needle with a pointed distal end arranged there within, the inserter being mounted to the medical device in a first position. When the inserter assembly is actuated (e.g. by release of a pre-actuated spring 2112) it moves the combined cannula and needle to an extended position in which the cannula engages the medical device. There after the needle is withdrawn and the inserter assembly with the needle can be arranged in a second position relative to the medical device. An advantage of this concept is that the complete inserter mechanism is not limited by the height of the device, but can have the necessary volume to cover a well-functioning insertion mechanism. A further advantage is that the soft catheter with the needle is hidden before use, and that the soft catheter is pulled out at the end of use when the device is disposed off. A yet further advantage is that the inserter folds down and integrates in a surface of the device after insertion.

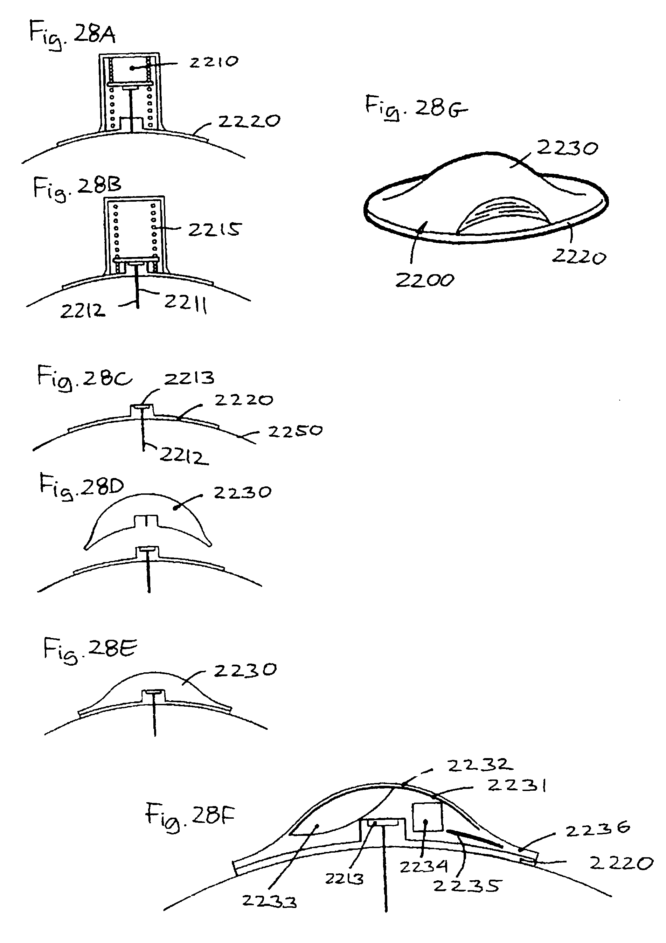

Figs. 28A-28G show a disposable inserter in combination with a compact soft patch pump assembly. A soft adhesive patch 2220 adapted to be arranged on a skin surface 2250 is supplied with a disposable spring-actuated 2215 inserter 2210 that is removed after insertion and pull-out of an insertion needle 2211, this leaving a cannula, e.g. a soft catheter 2212, in central position, where after a semi-flexible drug delivery device 2230 is placed on the soft adhesive patch, thereby forming a combined device 2200. The delivery device comprises an upper hard shield portion 2231, a flexible outer surface 2232, a reservoir 2233 in fluid communication with a pump assembly 2234 controlled by a processor mounted on a flexible PCB 2235, the pump assembly being adapted to engage a proximal portion 2213 of the cannula. The delivery device may be round in shape with flexible edges 2236 and moulded with a concave side towards the soft patch 2220, so that the flexible edges of the delivery device will be forced to stay tight to the flexible patch when the delivery device is flattened when it is attached corresponding to a central portion of the patch, this reducing the height of the edge portions of the device. An advantage of this invention is that the complete inserter mechanism is free of components of the delivery device and does not add weight and volume to the device in use. A further advantage is that the soft catheter with needle is hidden before use. A further advantage is that the soft adhesive patch is very flexible and skin-friendly. A yet further advantage is that the delivery device has a smooth surface that is less recognised on the body or under the clothes. A further advantage is that the "soft" shape of the delivery device reduces the risk of the delivery device being torn off by accident (see fig. 28G). A further advantage is that the delivery device (when generally round in shape) has, no general "direction". Also, the height of the edges is reduced as the edges of both patch and pump stay tight together. Further, the edge of the delivery device is forcing the edge of the patch towards the skin, this eliminating the well-known risk of an edge portion of the patch to peel off the skin. As appears, the central portion of the patch with the cannula and the attachment point to the delivery device will have to be properly attached to the skin surface of a subject as otherwise the delivery device would tend to lift the central area and thereby pull out the cannula.

## Claims

1. A medical device (500), comprising a transcutaneous device unit and a process unit, the transcutaneous device unit (502) comprising:
- a first housing (503),
- a lower surface adapted for application to a skin surface of a subject, and
- a transcutaneous device (530, 651),
the process unit comprising:
- a second housing (501) comprising a lower surface adapted to face towards the skin surface of the subject, and
- a process assembly (300, 580, 760),
wherein the first and second housings are adapted to be coupled to each other, **characterized in that** at least a portion of the lower surface of the second housing is free to move relative to the skin surface when the first unit is attached to the skin surface and the first and second housings are coupled to each other.

2. A medical device as in claim 1, wherein the transcutaneous device unit comprises a flexible patch portion (570) comprising an upper surface and a lower surface, the lower surface being adapted for application to the skin surface of the subject, and wherein the first and second housing are adapted to be coupled to each other with the lower surface of the second housing facing towards at least a portion of the upper surface of the patch portion, and
wherein at least a portion of the flexible patch portion facing towards the lower surface of the second housing is free to move relative thereto.

3. A medical device as in claim 1, wherein the transcutaneous device unit comprises a flexible patch portion comprising an upper surface and a lower surface, the lower surface being adapted for application to the skin surface of the subject, the first housing comprising a first coupling (511), wherein the process unit comprises a second coupling (506) arranged at a peripheral portion of the second housing, and wherein the first and second couplings can be connected to each other with the upper surface of the patch portion facing towards the lower surface of the second housing, and wherein the flexible patch portion facing towards the free lower surface of the second housing is free to move relative thereto.

4. A medical device as in claim 2 or 3, wherein the lower surface of the second housing and the upper surface of the patch is formed to prevent full engagement between the two surfaces when they are arranged against each other.

5. A medical device as in claim 4, wherein at least one of the two units comprises a raised portion (561) providing a free space between the two units when they are arranged against each other.

6. A medical device as in claim 2 or 3, wherein the patch portion facing towards the second housing comprises a cut-out portion

7. A medical device as in any of the previous claims, wherein the transcutaneous device unit comprises a flexible sheet member with an upper and a lower surface, the lower surface comprising an adhesive allowing the transcutaneous device unit to be attached to the skin surface of the subject, the first housing comprising a lower surface attached to the upper surface of the flexible sheet member.

8. A medical device as in claim 6, wherein a support (11, 561) extends from the first housing, the support being attached to the upper surface of the sheet member.

9. A medical device as in any of the previous claims, wherein the coupling is substantially rigid

10. A medical device as in any of the previous claims, wherein the transcutaneous device unit comprises a transcutaneous drug delivery device (530, 651), and the process unit comprises a reservoir (760) adapted to contain a fluid drug, and an expelling assembly (300) adapted for cooperation with the reservoir to expel fluid drug out of the reservoir and through the skin of the subject via the transcutaneous drug delivery device when the two units are coupled to each other.

11. A medical device as in any of the previous claims, wherein the transcutaneous device unit comprises a transcutaneous sensor device and the process unit is adapted to transmit and/or process data acquired via the sensor.

12. A medical device as in any of the previous claims, wherein the transcutaneous device in an initial state is arranged in a retracted position at least partially within the first housing, the transcutaneous device being adapted to be moved from the retracted position to an extended position, this allowing the transcutaneous device to be inserted into the subject when the transcutaneous device unit has been arranged on the skin surface of the subject.

## Patentansprüche

1. Medizinische Vorrichtung (500), umfassend eine transkutane Vorrichtungseinheit und eine Verarbeitungseinheit, wobei die transkutane Vorrichtungseinheit (502) Folgendes umfasst:
- ein erstes Gehäuse (503)
- eine untere Oberfläche, die zur Aufbringung auf einer Hautoberfläche eines Probanden angepasst ist, und
- eine transkutane Vorrichtung (530, 651),
wobei die Verarbeitungseinheit Folgendes umfasst:
- ein zweites Gehäuse (501), umfassend eine untere Oberfläche, die derart angepasst ist, dass sie die Hautoberfläche des Probanden zugewandt ist, und
- eine Verarbeitungsanordnung (300, 580, 760),
wobei das erste und zweite Gehäuse derart angepasst sind, dass sie aneinander gekoppelt werden, **dadurch gekennzeichnet, dass** zumindest ein Teil der unteren Oberfläche des zweiten Gehäuses frei ist, um sich in Bezug auf die Hautoberfläche zu bewegen, wenn die erste Einheit auf die Hautoberfläche aufgebracht wird, und das erste und zweite Gehäuse aneinander gekoppelt werden.

2. Medizinische Vorrichtung nach Anspruch 1, wobei die transkutane Vorrichtungseinheit einen flexiblen Pflasterteil (570) umfasst, umfassend eine obere Oberfläche und eine untere Oberfläche, wobei die untere Oberfläche zur Aufbringung auf die Hautoberfläche des Probanden angepasst ist, und wobei das erste und zweite Gehäuse derart angepasst sind, dass sie aneinander gekoppelt werden, wobei die untere Oberfläche des zweiten Gehäuses zumindest einem Teil der oberen Oberfläche des Pflasterteils zugewandt ist, und wobei zumindest ein Teil des der unteren Oberfläche des zweiten Gehäuses zugewandten flexiblen Pflasterteils frei ist, um sich in Bezug dazu zu bewegen.

3. Medizinische Vorrichtung nach Anspruch 1, wobei die transkutane Vorrichtungseinheit einen flexiblen Pflasterteil umfasst, umfassend eine obere Oberfläche und eine untere Oberfläche, wobei die untere Oberfläche zur Aufbringung auf die Hautoberfläche des Probanden angepasst ist,
wobei das erste Gehäuse eine erste Kopplung (511) umfasst, wobei die Verarbeitungseinheit eine an einem Umfangsteil des zweiten Gehäuses angeordnete zweite Kopplung (506) umfasst, und wobei die erste und zweite Kopplung miteinander verbunden werden können, wobei die obere Oberfläche des Pflasterteils der unteren Oberfläche des zweiten Gehäuses zugewandt ist, und wobei der der unteren Oberfläche des zweiten Gehäuses zugewandte flexible Pflasterteil frei ist, um sich in Bezug dazu zu bewegen.

4. Medizinische Vorrichtung nach Anspruch 2 oder 3, wobei die untere Oberfläche des zweiten Gehäuses und die obere Oberfläche des Pflasters gebildet sind, um einen vollständigen Eingriff zwischen den zwei Oberflächen zu verhindern, wenn sie gegeneinander angeordnet sind.

5. Medizinische Vorrichtung nach Anspruch 4, wobei zumindest eine der zwei Einheiten einen erhöhten Teil (561) aufweist, der einen Freiraum zwischen den zwei Einheiten bereitstellt, wenn sie gegeneinander angeordnet sind.

6. Medizinische Vorrichtung nach Anspruch 2 oder 3, wobei der dem zweiten Gehäuse zugewandte Pflasterteil einen Ausschnittsteil umfasst.

7. Medizinische Vorrichtung nach einem der vorangehenden Ansprüche,
wobei die transkutane Vorrichtungseinheit ein flexibles Folienelement mit einer oberen und einer unteren Oberfläche umfasst, wobei die untere Oberfläche einen Klebstoff umfasst, der es ermöglicht, dass die transkutane Vorrichtung auf der Hautoberfläche des Probanden angebracht wird, wobei das erste Gehäuse eine an der oberen Oberfläche des flexiblen Folienteils angebrachte untere Oberfläche umfasst.

8. Medizinische Vorrichtung nach Anspruch 6, wobei sich ein Träger (11, 561) vom ersten Gehäuse erstreckt, wobei der Träger an der oberen Oberfläche des Folienelements angebracht ist.

9. Medizinische Vorrichtung nach einem der vorangehenden Ansprüche,
wobei die Kopplung im Wesentlichen starr ist.

10. Medizinische Vorrichtung nach einem der vorangehenden Ansprüche,
wobei die transkutane Vorrichtungseinheit eine transkutane Arzneimittelabgabevorrichtung (530, 651) umfasst und die Verarbeitungseinheit einen zum Aufnehmen eines flüssigen Arzneimittels angepassten Behälter (760) und eine zur Zusammenarbeit mit dem Behälter angepasste Ausstoßanordnung (300) zum Ausstoßen eines flüssigen Arzneimittels aus dem Behälter und durch die Haut des Probanden über die transkutane Arzneimittelabgabevorrichtung, wenn die zwei Einheiten aneinander gekoppelt sind, umfasst.

11. Medizinische Vorrichtung nach einem der vorangehenden Ansprüche,
wobei die transkutane Vorrichtungseinheit eine transkutane Sensoreinheit umfasst und die Verarbeitungseinheit derart angepasst ist, dass sie über den Sensor angeforderte Daten überträgt und/oder verarbeitet.

12. Medizinische Vorrichtung nach einem der vorangehenden Ansprüche,
wobei die transkutane Vorrichtung in einem anfänglichen Zustand in einer zurückgezogenen Position zumindest teilweise innerhalb des ersten Gehäuses angeordnet ist, wobei die transkutane Vorrichtung derart angepasst ist, dass sie von der zurückgezogenen Position zu einer ausgefahrenen Position bewegt wird, wobei dies der transkutanen Vorrichtung ermöglicht, in den Probanden eingeführt zu werden, wenn die transkutane Vorrichtungseinheit auf der Hautoberfläche des Probanden angeordnet wurde.

## Revendications

1. Un dispositif médical (500) comprenant une unité de dispositif transcutané et une unité de traitement, l'unité de dispositif transcutané (502) comprenant :
- un premier boîtier (503),
- une surface inférieure apte à être appliquée sur une surface de peau d'un sujet, et
- un dispositif transcutané (530, 651),
l'unité de traitement comprenant :
- un second boîtier (501) comprenant une surface inférieure apte à être tournée en direction de la surface de peau du sujet, et
- un bloc de traitement (300, 580, 760),
dans lequel le premier et le second boîtiers sont aptes à être couplés l'un à l'autre, ***caractérisé en ce qu*'**au moins une partie de la surface inférieure du second boîtier est libre de se déplacer par rapport à la surface de la peau lorsque la première unité est fixée à la surface de la peau et que le premier et second boîtiers sont couplés l'un à l'autre.

2. Un dispositif médical selon la revendication 1, dans lequel l'unité de dispositif transcutané comprend une partie de timbre flexible (570) comprenant une surface supérieure et une surface inférieure, la surface inférieure étant apte à être appliquée sur la surface de peau du sujet, et dans lequel le premier et le second boîtiers sont aptes à être couplés l'un à l'autre avec la surface inférieure du second boîtier tournée vers au moins une partie de la surface supérieure de la partie de timbre, et dans lequel au moins une partie de la partie de timbre flexible tournée en direction de la surface inférieure du second boîtier est libre de se déplacer par rapport à celui-ci.

3. Un dispositif médical selon la revendication 1, dans lequel l'unité de dispositif transcutané comprend une partie de timbre flexible comprenant une surface supérieure et une surface inférieure, la surface inférieure étant apte à être appliquée sur la surface de peau du sujet, le premier boîtier comprenant un premier couplage (511), dans lequel l'unité de traitement comprend un second couplage (506) disposé en une partie périphérique du second boîtier, et dans lequel les premier et second couplages peuvent être connectés l'un à l'autre avec la surface supérieure de la partie de timbre tournée en direction de la surface inférieure du second boîtier, et dans lequel la partie de timbre flexible tournée en direction de la surface inférieure libre du second boîtier est libre de se déplacer par rapport à celui-ci.

4. Un dispositif médical selon la revendication 2 ou 3,
dans lequel la surface inférieure du second boîtier et la surface supérieure du timbre sont formées de manière à empêcher un contact complet entre les deux surfaces lorsqu'elles sont disposées l'une contre l'autre.

5. Un dispositif médical selon la revendication 4, dans lequel au moins l'une des deux unités comprend une partie surélevée (561) formant un espace libre entre les deux unités lorsqu'elles sont disposées l'une contre l'autre.

6. Un dispositif médical selon la revendication 2 ou 3,
dans lequel la partie de timbre tournée en direction du second boîtier comprend une partie découpée.

7. Un dispositif médical selon l'une des revendications précédentes, dans lequel l'unité de dispositif transcutané comprend un organe en feuille flexible avec une surface supérieure et inférieure, la surface inférieure comprenant un adhésif permettant de fixer l'unité de dispositif transcutané à la surface de peau du sujet, le premier boîtier comprenant une surface inférieure fixée à la surface supérieure de l'organe en feuille flexible.

8. Un dispositif médical selon la revendication 6, dans lequel un support (11, 561) s'étend depuis le premier boîtier, le support étant fixé à la surface supérieure de l'organe en feuille.

9. Un dispositif médical selon l'une des revendications précédentes, dans lequel le couplage est substantiellement rigide.

10. Un dispositif médical selon l'une des revendications précédentes, dans lequel l'unité de dispositif transcutané comprend un dispositif de délivrance de médicament transcutané (530, 651), et l'unité de traitement comprend un réservoir (760) apte à contenir un médicament fluide, et un bloc d'expulsion (300) apte à coopérer avec le réservoir pour expulser le médicament fluide hors du réservoir et au travers de la peau du sujet via le dispositif de délivrance de médicament transcutané lorsque les deux unités sont couplées l'une à l'autre.

11. Un dispositif médical selon l'une des revendications précédentes, dans lequel l'unité de dispositif transcutané comprend un dispositif capteur transcutané et l'unité de traitement est apte à émettre et/ou traiter des données acquises via le capteur.

12. Un dispositif médical selon l'une des revendications précédentes, dans lequel le dispositif transcutané dans un état initial est disposé en une position rétractée au moins partiellement à l'intérieur du premier boîtier, le dispositif transcutané étant apte à être déplacé de la position rétractée jusqu'à une position déployée, ceci permettant au dispositif transcutané d'être inséré à l'intérieur du sujet lorsque l'unité de dispositif transcutané a été disposé sur la surface de peau du sujet.
